# EUROPÄISCHE PATENTANMELDUNG

(11) **EP 2 676 680 A1**
(43) Veröffentlichungstag der Anmeldung: **25.12.2013**
(21) Anmeldenummer: 13174958.2
(22) Anmeldetag: 20.08.2008
(51) Int. Cl.: A61K 47/42, A61K 8/64, A61K 38/16, A01N 25/00, A01N 47/02, A61Q 19/00, A61K 9/00

(54) **Verwendung von Hydrophobin-Polipeptiden als Penetrationsverstärker**

(30) Priorität: 13.09.2007 EP 07116363; 27.11.2007 EP 07121650
(62) Teilanmeldung aus: 08803103.4
(71) Anmelder: BASF SE, 67056 Ludwigshafen (DE)
(72) Erfinder: Bollschweiler, Claus, 69118 Heidelberg (DE); Karos, Marvin, 68723 Schwetzingen (DE); Barg, Heiko, 67346 Speyer (DE); Subkowski, Thomas, 68526 Ladenburg (DE)
(74) Vertreter: Fitzner, Uwe

(57) **Zusammenfassung**

Die vorliegende Erfindung betrifft die Verwendung von Hydrophobin-Polypeptiden als Penetrationsverstärker.

## Beschreibung

Die vorliegende Erfindung betrifft die Verwendung von Hydrophobin-Polypeptiden als Penetrationsverstärker.

Penetrationsverstärker gewinnen in den letzten Jahren immer größere Bedeutung in verschiedenen Bereichen wie zum Beispiel als Bestandteil von kosmetischen oder pharmazeutischen Zusammensetzung, von Pflanzenschutzmitteln oder von Beschichtungsmitteln.

Transdermale Penetrationsverstärker sind aus der WO 93/002669 bekannt. Darin wird eine Kombination von polaren und unpolaren Penetrationsverstärkern in einer wirkstoffhaltiger Klebematrix in transdermalen therapeutischen Systemen beschrieben. Als polare Penetrationsverstärker werden mehrwertige Alkohole, als unpolare Penetrationsverstärker Fettsäureester genannt. In den beschriebenen transdermalen therapeutischen Systemen bewirken die Penetrationsverstärker eine Steigerung der Penetrationsgeschwindigkeit des Wirkstoffs, wobei es sich um in Wasser schwerlösliche oder unlösliche Steroidhormone handelt.

Bei den transdermalen therapeutischen Systemen wird die Barrierenfunktion des Stratum corneums durch Okklusioseffekte oder durch Penetrationsverstärker, wie die oben genannten oder Dimethylsulfoxid (DMSO), vorübergehend beeinträchtigt und so der Durchtritt für niedermolekulares Substanzen durch die Haut ermöglicht.

Weitere Penetrationsverstärker, die in therapeutischen Zubereitungen verwendet werden, sind zum Beispiel ein- oder mehrwertige Alkohole wie Ethanol, 1,2 Propandiol oder Benzylalkohol, gesättigte und ungesättigte Fettalkohole mit 8 bis 10 Kohlenstoffatomen wie Laurylalkohol oder Cetylalkohol, Kohlenwasserstoffe wie Mineralöl, Alkane, Ester, Azone , wie 1-Dodecylazacycloheptan-2-on, Propylenglykol, , Chitosan, gesättigte und ungesättigte Fettsäuren wie Stearinsäure oder Ölsäure, Fettsäureester mit bis zu 24 Kohlenstoffatomen oder Dicarbonsäurediester mit bis zu 24 Kohlenstoffatomen wie die Methylester, Ethylester, Isopropylester, Butylester, sec-Butylester, Isobutylester, tert.-Butylester oder Monoglycerinsäureester der Essigsäure, Capronsäure, Laurinsäure, Myristinsäure, Stearinsäure und Palmitinsäure, Phosphatderivate, wie Lecithin, Terpene, Harnstoff unsd seine Derivate und Ether wie Dimethylisosorbid und Diethylenglycolmonoethylether, Gallensalze, Polyethoxyethylene, EDTA, Nerolidol, Limonenoxide oder Phospholipide.

Weitere bekannte Penetrationsverstärker sind SDS (Sodium Dodecylsulfate), Dimethylformamid und N-Methylformamid.

Auch im Bereich des Pflanzenschutzes werdenPenetrationsverstärker verwendet um eine erleichterte Aufnahme von Pflanzenschutzmittel zu gewährleisten.

Hydrophobine sind kleine Proteine von etwa 100 bis 150 Aminosäuren, welche in filamentösen Pilzen, beispielsweise *Schizophyllum commune,* vorkommen. Sie weisen in aller Regel 8 Cystein-Einheiten auf. Hydrophobine können aus natürlichen Quellen iso-liert werden, können aber auch mittels gentechnischer Verfahren gewonnen werden, wie beispielsweise von WO 2006/082251 oder WO 2006/131564 offenbart.

Hydrophobine sind in einer wasserunlöslichen Form auf der Oberfläche von verschiedenen pilzlichen Strukturen, wie z.B. Lufthyphen, Sporen, Fruchtkörpern, verteilt. Die Gene für Hydrophobine konnten aus Ascomyzeten, Deuteromyzeten und Basidiomyzeten isoliert werden. Einige Pilze enthalten mehr als ein Hydrophobingen, z.B. Schizophyllum commune, Coprinus cinereus, Aspergillus nidulans. Offensichtlich sind verschiedene Hydrophobine in unterschiedlichen Stadien der pilzlichen Entwicklung involviert. Die Hydrophobine sind dabei vermutlich verantwortlich für unterschiedliche Funktionen (van Wetter et al., 2000, Mol. Microbiol., 36, 201-210; Kershaw et al. 1998, Fungal Genet. Biol, 1998, 23, 18-33).

Als biologische Funktion für Hydrophobine wird neben der Reduzierung der Oberflächenspannung von Wasser zur Generierung von Lufthyphen auch die Hydrophobisierung von Sporen beschrieben (Wösten et al. 1999, Curr. Biol., 19, 1985-88; Bell et al. 1992, Genes Dev., 6, 2382-2394). Weiterhin dienen Hydrophobine zur Auskleidung von Gaskanälen in Fruchtkörpern von Flechten und als Komponenten im Erkennungssystem von pflanzlichen Oberflächen durch pilzliche Pathogene (Lugones et al. 1999, Mycol. Res., 103, 635-640; Hamer & Talbot 1998, Curr. Opinion Microbiol., Band 1, 693-697).

Im Stand der Technik ist die Verwendung von Hydrophobinen für verschiedene Anwendungen vorgeschlagen worden.

WO 96/41882 schlägt die Verwendung von Hydrophobinen als Emulgatoren, Verdicker, oberflächenaktive Substanzen, zum Hydrophilieren hydrophober Oberflächen, zur Verbesserung der Wasserbeständigkeit hydrophiler Substrate, zur Herstellung von Öl-in-Wasser-Emulsionen oder von Wasser-in-Öl-Emulsionen vor. Weiterhin werden pharmazeutische Anwendungen wie die Herstellung von Salben oder Cremes sowie kosmetische Anwendungen wie Hautschutz oder die Herstellung von Haarshampoos oder Haarspülungen vorgeschlagen.

EP 1 252 516 offenbart die Beschichtung verschiedener Substrate, wie zum Beispiel Fenster, Objektiv, Biosensor, medizinische Vorrichtung, Behälter, Rahmen- oder Autokarosserie mit einer Hydrophobin enthaltenden Lösung bei einer Temperatur von 30 bis 80°C.

Weiterhin wurde beispielsweise die Verwendung als Demulgator (WO 2006/103251), als Verdunstungsverzögerer (WO 2006/128877) oder Verschmutzungsinhibitor (WO 2006/103215) vorgeschlagen.

Die US 20030217419A1 beschreibt die Verwendung des Hydrophobins SC3 aus *Schizophyllumg commune* für kosmetische Zubereitungen zur Behandlung von Therapien-Materialien. Dabei bilden sich kosmetische Depots, die mehrere Wäschen mit Shampoo widerstehen.

Die Verwendung von Hydrophobinen als Penetrationsverstärker ist bislang noch nicht bekannt.

Aufgabe der Erfindung war es, eine neue Verwendung für Hydrophobin bereitzustellen.

Zusätzlich sollte die Aufgabe gelöst werden, Wirkstoffen das Durchdringen von Phasengrenzen zu ermöglichen oder zu verbessern, für die bisher diese Phasengrenze impermeabel oder wenig permeabel war.

Weitere Aufgabe war es, die Konzentration der Wirkstoffe bei den Anwendungen herabzusetzen.

Ferner war es Aufgabe der vorliegenden Erfindung kosmetische und/oder pharmazeutische Zusammensetzungen enthaltend Hydrophobin als Penetrationsverstärker zur Verfügung zu stellen, die eine verbesserte Aufnahme der Wirkstoffe gewährleisten, insbesondere ohne Irritationen der Haut oder Schleimhäute zu verursachen.

Eine weitere Aufgabe der vorliegenden Erfindung war es Pflanzenschutzmittel enthaltend Hydrophobin als Penetrationsverstärker zur Verfügung zu stellen, die eine verbesserte Aufnahme der Wirkstoffe gewährleisten, insbesondere ohne Beschädigungen der behandelten Pflanzen oder Umweltschäden zu verursachen.

Die Aufgabe wird gelöst durch die Verwendung von Hydrophobin als Penetrationsverstärker.

Im Sinne der Erfindung sind die Begriffe "Penetrationsverstärker" und "Penetrationsförderer" synonym.

Zu der Erfindung ist im Einzelnen das Folgende auszuführen:
Penetration im Sinne der vorliegenden Erfindung ist das Durchdringen von Stoffen durch eine Phasengrenze.
Eine Phasengrenze im Sinne der vorliegenden Erfindung ist der Übergang von einer Phase in die benachbarte Phase.

Eine Phase im Sinne der vorliegenden Erfindung ist ein Bereich innerhalb dessen keine sprunghafte Änderung irgendeiner physikalischen Größe auftritt. Somit ändert sich bei dem Übergang von einer Phase in die benachbarte, also innerhalb einer Schicht von nur wenigen Moleküldurchmessern, mindestens eine physikalischen oder chemischen Eigenschaft gewählt aus der Gruppe bestehend aus Dichte, elektrische Eigenschaften, magnetische Eigenschaften, Brechungsindex, chemische Zusammensetzung, Kristallstruktur.

In einer Variante der vorliegenden Erfindung bedeutet Verstärkung der Penetration durch eine Phasengrenze, daß im Vergleich zu einer Kontrolle, welche die identischen chemischen, biologischen, und physikalischen Eigenschaften besitzt, und unter identischen chemischen biologischen und physikalischen Bedingungen oder Voraussetzungen, in der selber Zeit eine größere Menge Wirkstoffe die Phasengrenze durchdringt oder dieselbe Menge Wirkstoffe in einer kürzeren Zeit die Phasengrenze durchdringt.

In einer Variante der vorliegenden Erfindung bedeutet Verstärkung der Penetration oder die erhöhte Penetration, bzw. eine verstärkte Penetration von Wirkstoffen durch eine Phasengrenze, daß das Durchdringen von Phasengrenzen für Wirkstoffe möglich wird oder verbessert wird, für die bisher diese Phasengrenze impermeabel oder wenig permeabel war. Dies im Vergleich zu einer Kontrolle, welche die identischen chemischen, biologischen, und physikalischen Eigenschaften besitzt, und unter identischen chemischen biologischen und physikalischen Bedingungen oder Voraussetzungen, wobei in der selber Zeit eine größere Menge Wirkstoffe die Phasengrenze durchdringt oder dieselbe Menge Wirkstoffe in einer kürzeren Zeit die Phasengrenze durchdringt.

Penetrationsverstärker sind Substanzen, durch die die Penetration eines anderen Stoffes durch eine Phasengrenze verstärkt wird.

Unter dem Begriff "Hydrophobin" bzw. "Hydrophobine" im Sinne der vorliegenden Erfindung sollen im Folgenden Polypeptide der allgemeinen Strukturformel (I)

Xₙ-C¹ X₁₋₅₀-C²-X₀₋₅C³-X₁₋₁₀₀-C⁴-X₁₋₁₀₀-C⁵-X₁₋₅₀-C⁶-X₀₋₅-C⁷-X₁₋₅₀-C⁸-Xₘ (I)

verstanden werden, wobei X für jede der 20 natürlich vorkommenden Aminosäuren (Phe, Leu, Ser, Tyr, Cys, Trp, Pro, His, Gln, Arg, Ile Met, Thr, Asn, Lys, Val, Ala, Asp, Glu, Gly) stehen kann. Dabei können die Reste X jeweils gleich oder verschieden sein. Hierbei stellen die bei X stehenden Indizes jeweils die Anzahl der Aminosäuren in der jeweiligen Teilsequenz X dar, C steht für Cystein, Alanin, Serin, Glycin, Methionin oder Threonin, wobei mindestens vier der mit C benannten Reste für Cystein stehen, und die Indizes n und m stehen unabhängig voneinander für natürliche Zahlen zwischen 0 und 500, bevorzugt zwischen 15 und 300.

Die Polypetide gemäß der Formel (I) sind weiterhin durch die Eigenschaft charakterisiert, dass sie bei Raumtemperatur nach Beschichten einer Glasoberfläche eine Vergrößerung des Kontaktwinkels eines Wassertropfens von mindestens 8°, 10°, 20°, bevorzugt mindestens 25° und besonders bevorzugt 30° bewirken, jeweils verglichen mit dem Kontaktwinkel eines gleich großen Wassertropfens mit der unbeschichteten Glasoberfläche.

Die mit C¹ bis C⁸ benannten Aminosäuren sind bevorzugt Cysteine; sie können aber auch durch andere Aminosäuren ähnlicher Raumerfüllung, bevorzugt durch Alanin, Serin, Threonin, Methionin oder Glycin ersetzt werden. Allerdings sollen mindestens vier, bevorzugt mindestens 5, besonders bevorzugt mindestens 6 und insbesondere mindestens 7 der Positionen C¹ bis C⁸ aus Cysteinen bestehen. Cysteine können in den erfindungsgemäßen Proteinen entweder reduziert vorliegen oder miteinander Disulfidbrücken ausbilden. Besonders bevorzugt ist die intramolekulare Ausbildung von C-C Brücken, insbesondere die mit mindestens einer, bevorzugt 2, besonders bevorzugt 3 und ganz besonders bevorzugt 4 intramolekularen Disulfidbrücken. Bei dem oben beschriebenen Austausch von Cysteinen durch Aminosäuren ähnlicher Raumerfüllung werden vorteilhaft solche C-Positionen paarweise ausgetauscht, die intramolekulare Disulfidbrücken untereinander ausbilden können.

Falls in den mit X bezeichneten Positionen auch Cysteine, Serine, Alanine, Glycine, Methionine oder Threonine verwendet werden, kann sich die Nummerierung der einzelnen C-Positionen in den allgemeinen Formeln entsprechend verändern. Bevorzugt werden Hydrophobine der allgemeinen Formel (II)

Xₙ-C¹-X₃₋₂₅-C²-X₀₋₂-C³-X₅₋₅₀-C⁴-X₂₋₃₅-C⁵-X₂₋₁₅-C⁶-X₀₋₂-C⁷-X₃₋₃₅-C⁸-Xₘ (II)

zur Ausführung der vorliegenden Erfindung eingesetzt, wobei X, C und die bei X und C stehenden Indizes die obige Bedeutung haben, die Indizes n und m für Zahlen zwischen 0 und 350, bevorzugt 15 bis 300 stehen, sich die Proteine weiterhin durch die oben erwähnte Kontaktwinkeländerung auszeichnen, und es sich weiterhin bei mindestens 6 der mit C benannten Reste um Cystein handelt. Besonders bevorzugt handelt es sich bei allen Resten C um Cystein.

Besonders bevorzugt werden Hydrophobine der allgemeinen Formel (III)

Xₙ-C¹-X₅₋₉-C²-C³-X₁₁₋₃₉-C⁴-X₂₋₂₃-C⁵-X₅₋₉-C⁶-C⁷-X₆₋₁₈-C⁸-Xₘ (III)

eingesetzt, wobei X, C und die bei X stehenden Indizes die obige Bedeutung haben, die Indizes n und m für Zahlen zwischen 0 und 200 stehen, sich die Proteine weiterhin durch die oben erwähnte Kontaktwinkeländerung auszeichnen, und es sich bei mindestens 6 der mit C benannten Reste um Cystein handelt. Besonders bevorzugt handelt es sich bei allen Resten C um Cystein.

Bei den Resten Xₙ und Xₘ kann es sich um Peptidsequenzen handeln, die natürlicherweise auch mit einem Hydrophobin verknüpft sind. Es kann sich aber auch bei einem oder bei beiden Resten um Peptidsequenzen handeln, die natürlicherweise nicht mit einem Hydrophobin verknüpft sind. Darunter sind auch solche Reste Xₙ und/oder Xₘ zu verstehen, bei denen eine natürlicherweise in einem Hydrophobin vorkommende Peptidsequenz durch eine nicht natürlicherweise in einem Hydrophobin vorkommende Peptidsequenz verlängert ist.

Falls es sich bei Xₙ und/oder Xₘ um natürlicherweise nicht mit Hydrophobinen verknüpfte Peptidsequenzen handelt, sind derartige Sequenzen in der Regel mindestens 20, bevorzugt mindestens 35 Aminosäuren lang. Es kann sich beispielsweise um Sequenzen aus 20 bis 500, bevorzugt 30 bis 400 und besonders bevorzugt 35 bis 100 Aminosäuren handeln.

Ein derartiger, natürlicherweise nicht mit einem Hydrophobin verknüpfter Rest soll im Folgenden auch als Fusionspartner bezeichnet werden. Damit soll ausgedrückt werden, dass die Proteine aus mindestens einem Hydrophobinteil und einem Fusionspartnerteil bestehen können, die in der Natur nicht zusammen in dieser Form vorkommen. Fusions-Hydrophobine aus Fusionspartner und Hydrophobinteil sind beispielsweise in WO 2006/082251 (Seite 2, Zeile 18 bis Seite 5, Zeile 25), WO 2006/082253 (Seite 2, Zeile 20 bis Seite 6, Zeile 13)und WO 2006/131564 (Seite 2, Zeile 17 bis Seite 6, Zeile 26) offenbart worden.

Der Fusionspartnerteil kann aus einer Vielzahl von Proteinen ausgewählt werden. Es kann nur ein einziger Fusionspartner mit dem Hydrophobinteil verknüpft sein, oder es können auch mehrere Fusionspartner mit einem Hydrophobinteil verknüpft werden, beispielsweise am Aminoterminus (Xₙ) und am Carboxyterminus (Xₘ) des Hydrophobinteils. Es können aber auch beispielsweise zwei Fusionspartner mit einer Position (Xₙ oder Xₘ) des erfindungsgemäßen Proteins verknüpft werden.

Besonders geeignete Fusionspartner sind Proteine, die natürlicherweise in Mikroorganismen, insbesondere in E. coli oder Bacillus subtilis vorkommen. Beispiele für solche Fusionspartner sind die Sequenzen yaad (SEQ ID NO: 16 in WO 2006/082251), yaae (SEQ ID NO:18 in WO 2006/082251), Ubiquitin und Thioredoxin. Gut geeignet sind auch Fragmente oder Derivate dieser genannten Sequenzen, die nur einen Teil, beispielsweise 70 bis 99 %, bevorzugt 5 bis 50 %, und besonders bevorzugt 10 bis 40 % der genannten Sequenzen umfassen, oder bei denen einzelne Aminosäuren, bzw. Nukleotide gegenüber der genannten Sequenz verändert sind, wobei sich die Prozentangaben jeweils auf die Anzahl der Aminosäuren bezieht.

In einer weiterhin bevorzugten Ausführungsform weist das Fusion-Hydrophobin neben dem genannten Fusionspartner als eine der Gruppen Xₙ oder Xₘ oder als terminaler Bestandteil einer solchen Gruppe noch eine sogenannte Affinitätsdomäne (affinity tag / affinity tail) auf. Hierbei handelt es sich in prinzipiell bekannter Art und Weise um Ankergruppen, welche mit bestimmten komplementären Gruppen wechselwirken können und der leichteren Aufarbeitung und Reinigung der Proteine dienen können. Beispiele derartiger Affinitätsdomänen umfassen (His)ₖ- , (Arg)ₖ-, (Asp)ₖ-, (Phe)ₖ- oder (Cys)ₖ-Gruppen, wobei k im allgemeinen für eine natürliche Zahl von 1 bis 10 steht. Bevorzugt kann es sich um eine (His)ₖ-Gruppe handeln, wobei k für 4 bis 6 steht. Hierbei kann die Gruppe Xₙ und/oder Xₘ ausschließlich aus einer derartigen Affinitätsdomäne bestehen oder aber ein natürlicherweise oder nicht natürlicherweise mit einem Hydrophobin verknüpfter Rest Xₙ bzw. Xₘ wird um eine terminal angeordnete Affinitätsdomäne verlängert.

Die erfindungsgemäß verwendeten Hydrophobine sind Hydrophobine nach den Strukturfomeln (I), (II) und (III) sowie Fusions-Hydrophobine.

Die erfindungsgemäß verwendeten Hydrophobine können auch noch in ihrer Polypeptidsequenz modifiziert sein, beispielsweise durch Glycosilierung, Acetylierung oder auch durch chemische Quervernetzung beispielsweise mit Glutardialdehyd.

Eine Eigenschaft der erfindungsgemäß verwendeten Hydrophobine bzw. deren Derivaten ist die Änderung von Oberflächeneigenschaften, wenn die Oberflächen mit den Proteinen beschichtet werden. Die Änderung der Oberflächeneigenschaften lässt sich experimentell beispielsweise dadurch bestimmen, dass der Kontaktwinkel eines Wassertropfens vor und nach der Beschichtung der Oberfläche mit dem Protein gemessen wird und die Differenz der beiden Messungen ermittelt wird.

Die Durchführung von Kontaktwinkelmessungen ist dem Fachmann prinzipiell bekannt. Die Messungen beziehen sich auf Raumtemperatur sowie Wassertropfen von 5 µl und die Verwendung von Glasplättchen als Substrat. Die genauen experimentellen Bedingungen für eine beispielhaft geeignete Methode zur Messung des Kontaktwinkels sind im experimentellen Teil dargestellt. Unter den dort genannten Bedingungen besitzen die erfindungsgemäß verwendeten Fusionsproteine die Eigenschaft, den Kontaktwinkel um mindestens 20°, bevorzugt mindestens 25°, besonders bevorzugt mindestens 30°; 40°,45° insbesondere 50° zu vergrößern, jeweils verglichen mit dem Kontaktwinkel eines gleich großen Wassertropfens mit der unbeschichteten Glasoberfläche.

Besonders bevorzugte Hydrophobine zur Ausführung der vorliegenden Erfindung sind die Hydrophobine des Typs dewA, rodA, hypA, hypB, sc3, basf1, basf2. Diese Hydrophobine inklusive ihrer Sequenzen sind beispielsweise in WO 2006/82251 offenbart. Sofern nicht anders angegeben, beziehen sich die nachfolgend angegebenen Sequenzen auf in WO 2006/82251 offenbarten Sequenzen. Eine Übersichtstabelle mit den SEQ-ID-Nummern befindet sich in WO 2006/82251 auf Seite 20 (Zeile 1 bis Zeile 5).

Erfindungsgemäß insbesondere geeignet sind die Fusionsproteine yaad-Xa-dewA-his (SEQ ID NO: 20), yaad-Xa-rodA-his (SEQ ID NO: 22) oder yaad-Xa-basf1-his (SEQ ID NO: 24) mit den in Klammern angegebenen Polypeptidsequenzen sowie den dafür codierenden Nukleinsäuresequenzen, insbesondere den Sequenzen gemäß SEQ ID NO: 19, 21, 23. Besonders bevorzugt kann yaad-Xa-dewA-his (SEQ ID NO: 20) eingesetzt werden. Auch Proteine, die sich ausgehend von den in SEQ ID NO. 20, 22 oder 24 dargestellten Polypeptidsequenzen durch Austausch, Insertion oder Deletion von mindestens einer, bis hin zu 10, bevorzugt 5, besonders bevorzugt 5% aller Aminosäuren ergeben, und die die biologische Eigenschaft der Ausgangsproteine noch zu mindestens 50% besitzen, sind besonders bevorzugte Ausführungsformen. Unter biologischer Eigenschaft der Proteine wird hierbei die bereits beschriebene Änderung des Kontaktwinkels um mindestens 20°, bevorzugt mindestens 25°, besonders bevorzugt mindestens 30°, 40°,45° insbesondere 50° verstanden.

Besonders zur Ausführung der vorliegenden Erfindung geeignete Derivate sind von yaad-Xa-dewA-his (SEQ ID NO: 20), yaad-Xa-rodA-his (SEQ ID NO: 22) oder yaad-Xa-basf1-his (SEQ ID NO: 24) durch Verkürzung des yaad-Fusionspartners abgeleitete Derivate. Anstelle des vollständigen yaad-Fusionspartners (SEQ ID NO: 16) mit 294 Aminosäuren kann vorteilhaft ein verkürzter yaad-Rest eingesetzt werden. Der verkürzte Rest sollte aber zumindest 20, bevorzugt mindestens 35 Aminosäuren umfassen. Beispielsweise kann ein verkürzter Rest mit 20 bis 293, bevorzugt 25 bis 250, besonders bevorzugt 35 bis 150 und beispielsweise 35 bis 100 Aminosäuren eingesetzt werden. Ein Beispiel für ein derartiges Protein ist yaad40-Xa-dewA-his (SEQ ID NO: 26 in WO2007/014897)), welches einen auf 40 Aminosäuren verkürzten yaad-Rest aufweist.

Eine Spaltstelle zwischen dem Hydrophobin und dem Fusionspartner bzw. den Fusionspartnern kann dazu genutzt werden, den Fusionspartner abzuspalten und das reine Hydrophobin in underivatisierter Form freizusetzen (beispielsweise durch BrCN-Spaltung an Methionin, Faktor Xa-, Enterokinase-, Thrombin-, TEV-Spaltung etc.).

Die erfindungsgemäß als Penetrationsverstärker verwendeten Hydrophobine lassen sich chemisch durch bekannte Verfahren der Peptidsynthese, wie beispielsweise durch Festphasensynthese nach Merrifield herstellen.

Natürlich vorkommende Hydrophobine lassen sich aus natürlichen Quellen mittels geeigneter Methoden isolieren. Beispielhaft sei auf Wösten et. al., Eur. J Cell Bio. 63, 122-129 (1994) oder WO 96/41882 (Seite 23, Zeile 15 bis Seite 24, Zeile 8) verwiesen.

Ein gentechnisches Herstellverfahren für Hydrophobine ohne Fusionspartner aus *Talaromyces thermophilus* ist von US 2006/0040349 (Paragraphen [0071] bis [0090]) beschrieben.

Die Herstellung von Fusionsproteinen kann bevorzugt durch gentechnische Verfahren erfolgen, bei denen eine für den Fusionspartner und eine für den Hydrophobinteil codierende Nukleinsäuresequenz, insbesondere DNA-Sequenz, so kombiniert werden, dass in einem Wirtsorganismus durch Genexpression der kombinierten Nukleinsäuresequenz das gewünschte Protein erzeugt wird. Ein derartiges Herstellverfahren beispielsweise ist von WO 2006/082251 (Seite 6, Zeile 21 bis Seite 12, Zeile 37) oder WO 2006/082253 (Seite 5, Zeile 33 bis Seite 11, Zeile 13) offenbart. Die Fusionspartner erleichtern die Herstellung der Hydrophobine erheblich. Fusions-Hydrophobine werden bei den gentechnischen Verfahren mit deutlich besseren Ausbeuten produziert als Hydrophobine ohne Fusionspartner.

Die nach dem gentechnischen Verfahren von den Wirtsorganismen produzierten Fusions-Hydrophobine können in prinzipiell bekannter Art und Weise aufgearbeitet und mittels bekannter chromatographischer Methoden gereinigt werden.

In einer bevorzugten Ausführungsform kann das in WO 2006/082253, Seiten 11/12 (Seite 11, Zeile 15 bis Seite 11, Zeile 33) offenbarte, vereinfachte Aufarbeitungs- und Reinigungsverfahren eingesetzt werden.

Hierzu werden die fermentierten Zellen zunächst aus der Fermetationsbrühe abgetrennt, aufgeschlossen und die Zelltrümmer von den Einschlusskörpern (inclusion bodies) getrennt. Letzteres kann vorteilhaft durch Zentrifugieren erfolgen. Schließlich können die Einschlusskörper, beispielsweise durch Säuren, Basen und/oder Detergentien in prinzipiell bekannter Art und Weise aufgeschlossen werden, um die Fusions-Hydrophobine freizusetzen. Die Einschlusskörper mit den erfindungsgemäß verwendeten Fusion-Hydrophobinen können in der Regel schon unter Verwendung von 0,1 m NaOH innerhalb von ca. 1 h vollständig gelöst werden.

Die erhaltenen Lösungen können -ggf. nach Einstellen des gewünschten pH-Wertesohne weitere Reinigung zur Ausführung dieser Erfindung eingesetzt werden. Die Fusions-Hydrophobine können aus den Lösungen aber auch als Feststoff isoliert werden. Bevorzugt kann die Isolierung mittels Sprühgranulieren oder Sprühtrocknen erfolgen, wie in WO 2006/082253, (Seite 11, Zeile 35 bis Seite 12, Zeile 21) beschrieben. Die nach dem vereinfachten Aufarbeitungs- und Reinigungsverfahren erhaltenen Produkte umfassen neben Resten von Zelltrümmern in der Regel ca. 80 bis 90 Gew. % Proteine. Die Menge an Fusions-Hydrophobinen beträgt je nach Fusionskonstrukt und Fermentationsbedingungen in der Regel 30 bis 80 Gew. % bezüglich der Menge aller Proteine.

Die isolierten, Fusions-Hydrophobine enthaltenden Produkte können als Feststoffe gelagert werden und zum Einsatz in den jeweils gewünschten Medien gelöst werden.

Die Fusions-Hydrophobine können als solche oder auch nach Abspaltung und Abtrennung des Fusionspartners als "reine" Hydrophobine zur Ausführung dieser Erfindung verwendet werden. Eine Spaltung nimmt man vorteilhaft nach der Isolierung der Einschlusskörper und deren Auflösung vor.

Erfindungsgemäß werden die Hydrophobine als Penetrationsverstärker verwendet. In einer Ausführungsform wird Hydrophobin in Kombination mit mindestens einem weiteren Penetrationsverstärker verwendet, wobei mindestens ein weiterer Penetrationsverstärker ausgewählt wird aus der Gruppe: DMSO, SDS (Sodium Dodecylsulfate), Dimethylformamid ,N-Methylformamid ein- oder mehrwertige Alkohole wie Ethanol, 1,2 Propandiol oder Benzylalkohol, gesättigte und ungesättigte Fettalkohole mit 8 bis 10 Kohlenstoffatomen wie Laurylalkohol oder Cetylalkohol, Kohlenwasserstoffe wie Mineralöl, Alkane, Ester, Azone , wie 1-Dodecylazacycloheptan-2-on, Propylenglykol, Chitosan, gesättigte und ungesättigte Fettsäuren wie Stearinsäure oder Ölsäure, Fettsäureester mit bis zu 24 Kohlenstoffatomen oder Dicarbonsäurediester mit bis zu 24 Kohlenstoffatomen wie die Methylester, Ethylester, Isopropylester, Butylester, sec-Butylester, Isobutylester, tert.-Butylester oder Monoglycerinsäureester der Essigsäure, Capronsäure, Laurinsäure, Myristinsäure, Stearinsäure und Palmitinsäure, Phosphatderivate, wie Lecithin, Terpene, Harnstoff und seine Derivate und Ether wie Dimethylisosorbid und Diethylenglycolmonoethylether Gallensalze, Polyethoxyethylene, EDTA, Nerolidol, Limonenoxide oder Phospholipide.

In einer weiteren besonders bevorzugten Ausführungsform wird Hydrophobin als Pentrationsverstärker in Kombination mit DMSO oder Polyglykol verwendet.

In einer weiteren Ausführungsform der vorliegenden Erfindung wird Hydrophobin als Penetrationsverstärker in Kombination mit mindestens einem weiteren Penetrationsverstärker bei der Lederpflege und Lederbearbeitung verwendet.

In einer weiteren besonders bevorzugten Ausführungsform wird Hydrophobin als Penetrationsverstärker für Säuren und Basen, beispielsweise Carbonsäuren oder Ammoniak, Puffersysteme, Polymere, anorganische Partikel wie SiO₂ oder Silikate, Farbmittel wie beispielsweise Farbstoffe, Duftstoffe oder Biozide in Kombination mit mindestens einem weiteren Penetrationsverstärker bei der Lederpflege und Lederbearbeitung verwendet.

In einer weiteren Ausführung der vorliegenden Erfindung wird die Penetration durch eine Phasengrenze verstärkt.

In einer weiteren Ausführung wird dadurch die Penetration von Wirkstoffen gefördert.

In einer Ausführung der Erfindung sind unter Wirkstoffen alle Stoffe mit einer pharmazeutischen oder biologischen Wirkung zu verstehen. Wirkstoffe sind somit Verbindungen ausgewählt aus der Gruppe bestehend aus pharmazeutisch aktiven Verbindungen, therapeutisch wirksamen Verbindungen und biologisch aktiven Verbindungen, kosmetisch aktiven Verbindungen, Stoff zur Unterstützung eines kosmetischen Anspruchs (für Marketingzwecke) wie Perlprotein , die biochemische und/oder physiologische Prozesse in einem Organismus qualitativ und/oder quantitativ beeinflussen, das heißt fördern, oder überhaupt erst ermöglichen oder hemmen.

Wirkstoffe entfalten ferner in kleine Mengen eine große pharmazeutische, chemische, biologische oder physiologische Wirkung.

Dabei ist kleine Menge relativ zur Masse des Organismus zu sehen, den der Wirkstoff nach Durchdringen der Phasengrenze erreicht.

Dabei ergibt sich ein Quotient aus Masse des durch die Phasengrenze penetrierten Wirkstoffs zur Masse des Organismus ausgesucht aus der Gruppe bestehend aus den Intervallen: [1 ppt (1:10¹²) bis 10% (1:10)], [1 ppb (1:10⁹ bis 1% (1:100)], [1 ppt (1:10¹²) bis 1 ppb (1:10⁹], [1 ppt (1:10¹² bis 1:1000)], [1 ppb (1:10⁹ bis 1:1000)], [1 ppt (1:10¹² bis 1 ppm (1:10⁶)], [1 ppb (1:10⁹ bis 1 ppm (1:10⁶)], [1 ppm (1:10⁶ bis 1:1000)], [(1:1000) bis 1% (1:100)].

In einer Variante der vorliegenden Erfindung ist ein Organismus ausgesucht aus der Gruppe bestehend aus einzelnen Individuen ausgewählt aus dem Reich der Protisten, Bakterien, Pilzen, Pflanzen oder Tieren und sowie Teilen davon wie Zellen und Zellgeweben.

In einer weiteren Variante der vorliegenden ist ein Organismus ein toter Organismus oder Teile davon, wie zum Beispiel Haut für die Herstellung von Leder.

Durch die Anwendung von Hydrophobin als Penetrationsverstärker kann die Verstärkung der Penetration des Wirkstoff im Vergleich zur Kontrolle 0,5; 0,6; 0,7;0,9 oder 1 % betragen. Vorteilhaft ist eine Verstärkung der Penetration um 2,3,4,5,6,7,8,9 oder 10 %, besonders vorteilhaft ist eine Verstärkung der Penetration um 11,12,13,14 oder 15 %, ganz besonders vorteilhaft ist eine Verstärkung der Penetration um 16,17,18,19 oder 20 % oder mehr %.

Ein weiterer Gegenstand der vorliegenden Erfindung ist die Verwendung von Hydrophobin zur Herstellung eines Mittels zur verbesserten Aufnahme von Wirkstoffen bei topischer Anwendung.

Ein weiteres Anwendungsgebiet für die erfindungsgemäße Verwendung von Hydrophobin als Penetrationsverstärker ist bei der Herstellung von dermatologischen Zubereitungen.

In einer Ausführung wird somit Hydrophobin verwendet in einem Verfahren zur Herstellung von halbfesten Arzneiformen oder kosmetischen Zubereitungen, ausgesucht aus der Gruppe bestehend aus Salbe, Creme, Gel und Paste.

Die halbfesten Arzneiformen werden zubereitet wie zum Beispiel beschrieben in "Arzneiformelehre" von Ursula Schöffling, 4. Auflage, Deutsche Apotheker Verlag, 2003, Seiten 353 bis 392.

Die Zubereitungen enthalten neben den dort beschriebenen Hilfsstoffen Hydrophobin in einem Anteil ausgewählt aus der Gruppe bestehend aus 0,000001 bis 10 Gew.-%, 0,0001 bis 10 Gew.-%, 0,001 bis 10 Gew.-%, 0,01 bis 10 Gew.-%0,1 bis 10 Gew.-% und 1 bis 10 Gew.-% , sowie Wirkstoffe in einem Anteil ausgewählt aus der Gruppe bestehend aus 0,000001 bis 10 Gew.-%, 0,0001 bis 10 Gew.-%, 0,001 bis 10 Gew.-%, 0,01 bis 10 Gew.-% 0,1 bis 10 Gew.-% und 1 bis 10 Gew.-%

Ein weiteres Anwendungsgebiet für die erfindungsgemäße Verwendung von Hydrophobin als Penetrationsverstärker ist bei der Herstellung von Mitteln für den therapeutischen oder prophylaktischen Einsatz bei bestimmten Erkrankungen der Haut und der Schleimhäute. Anwendungsgebiete hierfür sind insbesondere:
- virale Erkrankungen (z.B. Herpes, Coxsackie, Varicella zoster, Cytomegalovirus etc)
- bakterielle Erkrankungen (z.B. TB, Syphilis etc.)
- pilzliche Erkrankungen (z.B. Candida, Cryptococcus, Histoplasmosis, Aspergillus, Mucormycosis etc.)
- Tumorerkrankungen (z.B. Melanome, Adenome etc.)
- Autoimmunerkrankungen (z.B. PEMPHIGUS VULGARIS, BULLOUS PEMPHI-GOID, SYSTEMIC LUPUS ERYTHEMATOSIS etc.)
- Sonnenbrand
- parasitärer Befall (z.B. Zecken, Milben, Flöhe etc.)
- Insektenkontakt (z.B. blutsaugende Insekten wie Anopheles etc.)

In einer Ausführung der Erfindung liegen die Zubereitungen für die oben genannten Anwendungen als Aerodispersionen vor, wie zum Beispiel beschrieben in "Arzneiformelehre" von Ursula Schöffling, 4. Auflage, Deutsche Apotheker Verlag, 2003, Seiten 336 bis 352.

In einer Ausführung der Erfindung liegen die Zubereitungen für die oben genannten Anwendungen als Freisetzungssysteme vor, ausgesucht aus der Gruppe bestehen aus Nanaopartikel, Nanosuspensionen, Liposomen, Microemulsion und bioadhäsive Zubereitungsformen wie zum Beispiel beschrieben in "Arzneiformelehre" von Ursula Schöffling, 4. Auflage, Deutsche Apotheker Verlag, 2003, Seiten 468 bis 471.

Die Zubereitungen enthalten neben den dort beschriebenen Hilfsstoffen Hydrophobin in einem Anteil ausgewählt aus der Gruppe bestehend aus 0,000001 bis 10 Gew.-%, 0,0001 bis 10 Gew.-%, 0,001 bis 10 Gew.-%, 0,01 bis 10 Gew.-%. 0,1 bis 10 Gew.-% und 1 bis 10 Gew.-% , sowie Wirkstoffe in einem Anteil ausgewählt aus der Gruppe bestehend aus 0,000001 bis 10 Gew.-%, 0,0001 bis 10 Gew.-%, 0,001 bis 10 Gew.-%, 0,01 bis 10 Gew.-% , 0,1 bis 10 Gew.-% und 1 bis 10 Gew.-% .....????.

Ein weiteres Anwendungsgebiet für die erfindungsgemäße Verwendung von Hydrophobin als Penetrationsverstärker ist bei der Herstellung von Membranen, Matrix oder Pflaster enthaltend Wirkstoffe, z.B. ausgesucht aus der Gruppe bestehen aus transdermalen therapeutischen Systemen TTS.

Diese werden zubereitet wie zum Beispiel beschrieben in "Arzneiformelehre" von Ursula Schöffling, 4. Auflage, Deutsche Apotheker Verlag, 2003, Seiten 462 bis 468.

Die Zubereitungen enthalten neben den dort beschriebenen Hilfsstoffen Hydrophobin in einem Anteil ausgewählt aus der Gruppe bestehend aus 0,000001 bis 30 Gew.-%, 0,0001 bis 30 Gew.-%, 0,001 bis 30 Gew.-%, 0,01 bis 30 Gew.-% 0,1 bis 30 Gew.-% und 1 bis 30 Gew.-% , sowie Wirkstoffe in einem Anteil ausgewählt aus der Gruppe bestehend aus 0,000001 bis 30 Gew.-%, 0,0001 bis 30 Gew.-%, 0,001 bis 30 Gew.-%, 0,01 bis 30 Gew.-%, 0,1 bis 30 Gew.-%, 1 bis 30 Gew.-% und 0,1 bis 50 Gew.-%, 1 bis 50 Gew.-% .

Einer weitere Ausführung für die erfindungsgemäße Verwendung von Hydrophobin als Penetrationsverstärker ist bei der Herstellung von kosmetischen Zubereitungen.

In einer Variante handelt es sich dabei um haarkosmetische, hautkosmetische oder zahnkosmetische Zubereitungen.

In den erfindungsgemäß genannten Zubereitungen oder Zusammensetzungen können in einer Ausführung der vorliegenden Erfindung Effektormoleküle als Wirkstoffe eingesetzt werden.

Als Effektormoleküle werden im folgenden Moleküle verstanden, die eine bestimmte, vorhersehbare Wirkung aufweisen. Dies können entweder proteinartige Moleküle sein, wie Enzyme oder auch nicht-proteinogene Moleküle wie Farbstoffe, Lichtschutzmittel, Vitamine und Fettsäuren, oder Metallionen- enthaltende Verbindungen.

Unter den proteinartigen Effektormolekülen sind Enzyme, Peptide und Antikörper, bevorzugt.

Unter den Enzymen sind folgende als Effektormoleküle bevorzugt: Oxidasen, Peroxidasen, Proteasen, Tyrosinasen, metallbindende Enzyme, Lactoperoxidase, Lysozym, Amyloglycosidase, Glucoseoxidase, Superoxiddismutase, Photolyase, Calalase.

Gut geeignet als proteinartige Effektormoleküle sind auch Hydrolysate von Proteinen aus pflanzlichen und tierischen Quellen, beispielsweise Hydrolysate von Proteinen marinen Ursprungs oder Seidenhydrolysate.

Besonders gut geeignet sind definierte Peptide, die zum Anti-Aging eingesetzt werden, wie Matrixyl (INCI Name Glycerin-Water-Butylene Glycol-Carbomer-Polysorbate 20-Palmitoyl Pentapeptide-4), Argireline (INCI Name Aqua, Acety-Hexapeptide-3), Rigin (INCI Name Water (and)-Glycerin (and) Steareth-20 (and) Palmitoyltetrapeptide-7, Eyeliss (INCI Name Water-Glycerin-Hespiridin Methyl Chalcone-Steareth-20-Dipeptide-2-Palmitoyl Tetrapeptide-7) Regu-Age (INCI Name Oxido Reductases-Soy Peptides-Hydrilyzed Rice Bran Extract) und Melanostatin-5 (INCI Name Aqua-dextran-Nonapetide-1).

Unter den nicht-proteinartigen Effektormolekülen sind Farbstoffe beispielsweise semipermanente Farbstoffe oder Oxidationsfarbstoffe bevorzugt. Als Farbstoffe sind alle gängigen Haarfarbstoffe für die erfindungsgemässen Moleküle geeignet. Geeignete Farbstoffe sind dem Fachmann aus Handbüchern der Kosmetik beispielsweise Schrader, Grundlagen und Rezepturen der Kosmetika, Hüthig Verlag, Heidelberg, 1989, ISBN 3-7785-1491-1 bekannt.

Weiterhin sind Antioxidantien als Effektormoleküle bevorzugt. Antioxidantien, die auch als Radikalfänger bezeichnet werden, sind in der Lage, sog. freie Radikale zu neutralisieren. Dies sind aggressive Verbindungen, die physiologischerweise bei zahlreichen Stoffwechselvorgängen und der Energiegewinnung entstehen. Sie sind für Abwehrreaktionen des Körpers wichtig, können aber auch Schäden an der Erbsubstanz (DNA), den Zellmembranen und Körpereiweißen hervorrufen. Diese Schädigungen können zu vorzeitiger Gewebealterung, Gewebetod und Krebs führen. Zu den Antioxidatien zählen Carotinoide Ascorbinsäure (Vitamin C, E 300) wie auch Natrium-L-Ascorbat (E 301) und Calcium-L-Ascorbat (E 302); Ascorbylpalmitat (E 304); Butylhydroxyanisol (E 320); Butylhydroxytoluol (E 321); Calcium-Dinatrium-EDTA (E 385); Gallat wie auch Propylgallat (E 310), Octylgallat (E 311) und Dodecylgallat (Laurylgallat) (E 312); Isoascorbinsäure (E 315) wie auch Natriumisoascorbat (E 316); Lecithin (E 322); Milchsäure (E 270); Mehrfach-Phosphate wie Diphosphate (E 450), Triphosphate (E 451) und Polyphosphate (E 452); Schwefeldioxid (E 220) wie auch Natriumsulfit (E 221), Natriumbisulfit (E 222), Natriumdisulfit (E 223), Kaliumsulfit (E 224), Calciumsulfit (E 226), Calciumhydrogensulfit (E 227) und Kaliumbisulfit (E 228); Selen; Tocopherol (Vitamin E, E 306) wie auch Alpha-Tocopherol (E 307), Gamma-Tocopherol (E 308) und Delta-Tocopherol (E 309); Zinn-II-Chlorid (E 512); Zitronensäure (E 330) wie auch Natriumcitrat (E 331) und Kaliumcitrat (E 332); L-Gluthation, L-Cystein, Polyphenole, Flavonoide, Phytoöstrogene, Gluthation und die antoxidativen Enzyme Superoxiddismutase, Glutathionperoxidase und Katalase.

Erfindungsgemäß werden als Antioxidatien mindestens eine Verbindung aus der oben genannten Gruppe von Antioxidatien ausgewählt.

Weitere geeignete Effektormoleküle sind Carotinoide. Unter Carotinoiden sind erfindungsgemäß folgende Verbindungen zu verstehen: beta-Carotin, Lycopin, Lutein, Astaxanthin, Zeaxanthin, Cryptoxanthin, Citranaxanthin, Canthaxanthin, Bixin, beta-Apo-4-carotinal, beta-Apo-8-carotinal, beta-Apo-8-carotinsäureester, einzeln oder als Mischung. Bevorzugt verwendete Carotinoide sind beta-Carotin, Lycopin, Lutein, Astaxanthin, Zeaxanthin, Citranaxanthin und Canthaxanthin.

Unter Retinoide sind im Rahmen der vorliegenden Erfindung Vitamin A Alkohol (Retinol) und seine Derivate wie Vitamin A Aldehyd (Retinal), Vitamin A Säure (Retinsäure) und Vitamin A Ester (z.B. Retinylacetat, Retinylpropionat und Retinylpalmitat) gemeint. Der Begriff Retinsäure umfasst dabei sowohl all-trans Retinsäure als auch 13-cis Retinsäure. Die Begriffe Retinol und Retinal umfassen bevorzugt die all-trans Verbindungen. Als bevorzugtes Retinoid verwendet man für die erfindungsgemäßen Suspensionen all-trans-Retinol, im folgenden als Retinol bezeichnet.

Weitere bevorzugte Effektormoleküle sind Vitamine, insbesondere Vitamin A und deren Ester.

Vitamine sind essentielle organische Verbindungen, die entweder im tierischen und menschlichen Organismus nicht oder nur in unzureichenden Mengen synthetisiert werden. Aufgrund dieser Definition wurden 13 Komponenten oder Gruppen von Komponenten als Vitamine klassifiziert. Zu den fettlöslichen Vitaminen gehören Vitamin A (Retinole), Vitamin D (Calciferole), Vitamin E (Tocopherole, Tocotrienole) und Vitamin K (Phylloquinone). Zu den wasserlöslichen Vitaminen gehören Vitamin B₁ (Thiamin), Vitamin B₂ (Riboflavin), Vitamin B₆ (Pyridoxalgruppe), Vitamin B₁₂ (Cobalamine), Vitamin C (L-Ascobinsäure), Panthotensäure, Biotin, Folsäure und Niacin.

Vitamine, Provitamine und Vitaminvorstufen aus den Gruppen A, C, E und F, insbesondere 3,4-Didehydroretinol, beta-Carotin (Provitamin des Vitamin A), Ascorbinsäure (Vitamin C), sowie die Palmitinsäureester, Glucoside oder Phosphate der Ascorbinsäure, Tocopherole, insbesondere a-Tocopherol sowie seine Ester, z.B. das Acetat, das Nicotinat, das Phosphat und das Succinat; weiterhin Vitamin F, worunter essentielle Fettsäuren, besonders Linolsäure, Linolensäure und Arachidonsäure, verstanden werden;

Vitamin E ist ein Sammelbegriff für eine Gruppe von (bis heute) acht fettlöslichen Substanzen mit antioxidativen und nicht-antioxidativen Wirkungen. Vitamin E ist Bestandteil aller Membranen tierischer Zellen, wird jedoch nur von photosynthetisch aktiven Organismen wie Pflanzen und Cyanobakterien gebildet. Vier der acht bekannten Vitamin E-Formen werden Tocopherole (alpha-Tocopherol, beta-Tocopherol, gamma-Tocopherol und delta-Tocopherol) genannt. Die anderen bisher bekannten vier Formen von Vitamin E werden Tocotrienole ((alpha-Tocotrienol, beta-Tocotrienol, gamma-Tocotrienol und delta-Tocotrienol) genannt. Ferner können auch Derivate dieser Substanzen wie alpha-Tocopherylacetat vorteilhaft sein.

Vitamin A und seine Derivate und Provitamine zeigen vorteilhafterweise einen besonderen hautglättenden Effekt.

Zu den erfindungsgemäß bevorzugt einzusetzenden Vitaminen, Provitaminen oder Vitaminvorstufen der Vitamin B-Gruppe oder deren Derivaten sowie den Derivaten von 2-Furanon gehören unter anderem:
Vitamin B₁ , Trivialname Thiamin, chemische Bezeichnung 3-[(4'-Amino-2'-methyl-5'-pyrimidinyl) methyl]-5-(2-hydroxyethyl)-4-methylthiazoliumchlorid.
Vitamin B₂, Trivialname Riboflavin, chemische Bezeichung 7,8-Dimethyl-10-(1-D-ribityl)-benzo[g]pteridin-2,4(3H,10H)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt.
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid.
Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können zusätzlich zu Pantothensäure oder Panthenol auch Derivate des 2-Furanon eingesetzt werden. Besonders bevorzugte Derivate sind die auch im Handel erhältlichen Substanzen Dihydro-3 hydroxy-4,4-dimethyl-2(3H)-furanon mit dem Trivialnamen Pantolacton (Merck), 4 Hydroxymethyl-g-butyrolacton (Merck), 3,3-Dimethyl-2-hydroxy-g-butyrolacton (Aldrich) und 2,5- Dihydro-5-methoxy-2-furanon (Merck), wobei ausdrücklich alle Stereoisomeren eingeschlossen sind.
Vitamin B₆, wobei man hierunter keine einheitliche Substanz, sondern die unter den Trivialnamen Pyridoxin, Pyridoxamin und Pyridoxal bekannten Derivate des 5 Hydroxymethyl-2-methylpyridin-3-ols versteht.
Vitamin B₇ (Biotin), auch als Vitamin H oder "Hautvitamin" bezeichnet. Bei Biotin handelt es sich um (3aS,4S, 6aR)-2-Oxohexahydrothienol[3,4-d]imidazol-4-valeriansäure.

Panthenol, Pantolacton, Nicotinsäureamid sowie Biotin sind erfindungsgemäß ganz besonders bevorzugt.

Erfindungsgemäß können geeignete Derivate (Salze, Ester, Zucker, Nukleotide, Nukleoside, Peptide und Lipide) genutzt werden.

Als lipophile, öllösliche Antioxidantien aus dieser Gruppe sind Tocopherol und dessen Derivate, Gallussäureester, Flavonoide und Carotinoide sowie

Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt.

Triterpene, insbesondere Triterpensäuren wie Ursolsäure, Rosmarinsäure, Betulinsäure, Boswelliasäure und Bryonolsäure.

Weitere bevorzugte Effektormoleküle sind, bevorzugt niedrig dosierte, Fruchtsäuren (Alphahydroxysäuren) wie zum Beispiel Apfelsäure, Zitronensäure, Milchsäure, Weinsäure, Glykolsäure.

Erfindungsgemäß werden als Effektormoleküle mindestens eine Verbindung aus der oben genannten Gruppe von Fruchtsäuren ausgewählt.

Diese können in Konzentrationen von 0.1 % bis 35%, bevorzugt 0.1 % bis 10%, insbesondere 1 % bis 10%, 1% bis 5% vorliegen.

Weitere bevorzugte Effektormoleküle sind Harnstoff und Derivate davon.

Diese können in Konzentrationen von 0.1 % bis 25%, bevorzugt 0.1 % bis 10%, insbesondere 1 % bis 10%, 1 % bis 5% vorliegen.

In eine Ausführung der vorliegende Erfindung werden die Effektormoleküle mit der Hydrophobin-Polypeptidsequenz verknüpft.

Die Effektormoleküle sind mit einer Hydrophobin-Polypeptidsequenz verbunden. Die Verbindung zwischen Effektormoleküle und Hydrophobin-Polypeptidsequenz kann sowohl kovalent als auch auf ionische oder van-der Waals Wechselwirkungen beruhen.

Bevorzugt ist eine kovalente Verknüpfung. Dies kann beispielsweise über die Seitenketten der Hydrophobin-Polypeptidsequenz erfolgen, insbesondere über Aminofunktionen oder Carboxylatfunktionen oder Thiolfunktionen. Bevorzugt ist eine Verknüfung über die Aminofunktionen von einem oder mehreren Lysinresten, einer oder mehrerer Thiolgruppe von Cysteinresten oder über die N-terminale oder C-terminale Funktion des Hydrophobin-Polypeptids. Die Verknüpfung der Effektormoleküle mit der Hydrophobin-Polypeptidsequenz kann entweder direkt, d.h. als kovalente Verknüpfung zweier in dem Effektormolekül und der Hydrophobin-Polypeptidsequenz bereits vorhandener chemischer Funktionen erfolgen, beispielsweise eine Aminofunktion der Hydrophobin-Polypeptidsequenz wird mit einer Carboxylatfunktion des Effektormoleküls zum Säureamid verknüpft. Die Verknüpfung kann aber auch über einen sogenannten Linker, d.h. einem mindestens bifunktionellen Molekül erfolgen, der mit einer Funktion der Hydrophobin-Polypeptidsequenz eine Bindung eingeht und mit einer anderen Funktion des Effektormoleküls verknüpft wird. Falls das Effektormolekül ebenfalls aus einer Polypeptidsequenz besteht, kann die Verknüpfung von Effektormoleküle und Hydrophobin-Polypeptidsequenz durch ein sogenanntes Fusionsprotein erfolgen, d.h. eine durchgängige Polypeptidsequenz, die aus den beiden Teilsequenzen d.h. aus Effektormoleküle und Hydrophobin-Polypeptidsequenz besteht.

Es können zwischen Effektormolekülen und Hydrophobin-Polypeptidsequenz auch noch sogenannte Spacerelemente eingebaut werden, beispielsweise Polypeptidsequenzen, die eine potentielle Spaltstelle für eine Protease, Lipase, Esterase, Phosphatase, Hydrolase besitzen oder Polypeptidsequenzen, die eine leichte Aufreinigung des Fusionsproteins gestatten, beispielsweise sogenannte Histags, d.h. Oligohistidinreste.

Die Verknüpfung im Falle eines nicht-proteinartigen Effektormoleküls mit der Hydrophobin-Polypeptidsequenz erfolgt bevorzugt durch funktionalisierbare Reste (Seitengruppen) am Hydrophobin-Polypeptid, die mit einer chemischen Funktion des Effektormoleküls eine kovalente Verbindung eingehen.

Bevorzugt ist hier die Bindungsknüpfung über eine Amino-, Thiol- oder Hydroxyfunktion des Hydrophobin-Polypeptids, die beispielsweise mit einer Carboxylfunktion des Effektormoleküls , ggf. nach Aktivierung, eine entsprechende Amid-, Thioester oder Esterbindung eingehen können.

Eine weitere bevorzugte Verknüpfung der Hydrophobin-Polypeptidsequenz mit einem Effektormolekül ist die Verwendung eines massgeschneiderten Linkers. Ein solcher Linker hat zwei oder mehr sogenannte Ankergruppen mit denen er die Hydrophobin-Polypeptidsequenz und ein oder mehrere Effektormoleküle verknüpfen kann. Beispielsweise kann eine Ankergruppe für Hydrophobin-Peptid eine Thiolfunktion sein, mittels derer der Linker mit einem Cysteinrest des Hydrophobin-Polypeptids eine Disulfidbindung eingehen kann. Eine Ankergruppe für das Effektormolekül kann beispielsweise eine Carboxylfunktion sein, mittels derer der Linker mit einer Hydroxylfunktion des Effektormoleküls eine Esterbindung eingehen kann.

Die Verwendung solcher massgeschneiderter Linker erlaubt die genaue Anpassung der Verknüpfung an das gewünschte Effektormolekül. Ausserdem ist es dadurch möglich, mehrere Effektormoleküle mit einer Hydrophobin-Polypeptidsequenz definiert zu verknüpfen.

Der verwendete Linker richtet sich nach der zu koppelnden Funktionalität. Geeignet sind z.B. Moleküle, die zu Hydrophobin-Polypeptiden koppeln mittels Sulfhydryl-reaktiven Gruppen, z.B. Maleimide, Pydridyldisulfide, alpha -Haloacetyle, Vinylsulfon und zu Effektormolekülen mittels
- Sulfhydryl-reaktiven Gruppen z.B. Maleimide, Pydridyldisulfide, alpha-Haloacetyle, Vinylsulfone) Amin-reaktive Gruppen (z.B. Succinimidylester, Carbodiimide, Hydroxymethyl- Phosphin, Imidoester, PFP-Ester etc.)
- Zucker bzw. oxidierte Zucker-reaktive Gruppen (z.B. Hydrazide etc.)
- Carboxy-reaktive Gruppen (z.B. Carbodiimide etc.)
- Hydroxyl-reaktive Gruppen (z.B. Isocyanate etc.)
- Thymin-reaktive Gruppen (z.B. Psoralen etc.)
- unselektive Gruppen (z.B. Arylazide etc.)
- photoaktivierbare Gruppen (z.B. Perfluorphenylazid etc.)
- Metallkomplexierenden Gruppen (z.B. EDTA, Hexahis, Ferritin)
- Antikörper und -fragmente (z.B. single-chain antibodies, F(ab)-Fragmente von Antikörpern, katalytische Antikörper).

Alternativ kann eine direkte Kopplung zwischen Wirk-/Effektstoff und der Keratin-Bindedomäne z.B. mittels Carbodiimiden, Glutardialdehyd oder anderen, dem Fachmann bekannten Crossiinkern durchgeführt werden.

Der Linker kann stabil, thermospaltbar, photospaltbar oder auch enzymatisch spaltbar sein (besonders durch Lipasen, Esterasen, Proteasen, Phosphatasen, Hydrolasen etc.). Entsprechende chemische Strukturen sind dem Fachmann bekannt und werden zwischen die Molekülteile integriert.

Beispiele für enzymatisch spaltbare Linker, die bei den erfindungsgemässen Molekülen eingesetzt werden können, sind beispielsweise in WO 98/01406 (Seite 3, Zeile 30 bis Seite 23, Zeile 9) genannt, auf deren gesamten Inhalt hiermit ausdrücklich Bezug genommen wird.

Die erfindungsgemässen Zubereitungen enthaltend Hydrophobin als Penetrationsverstärker besitzen ein weiteres Anwendungsgebiet in der Humankosmetik, insbesondere der Haut- und Haarpflege, Zahnpflege, der Tierpflege, der Lederpflege und Lederbearbeitung.

Bevorzugt werden die Zubereitungen für die Haut-, Nagel-, Zahn- und Haarkosmetik angewendet. Sie erlauben eine hohe Konzentration und lange Wirkdauer von haut-, nagel-, zahn- und haarpflegenden oder haut-, nagel-, zahn- und haarschützenden Effektorstoffen.

Geeignete Hilfs- und Zusatzstoffe für die Herstellung von haarkosmetischen, zahnkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1 entnommen werden.

Nach einer weiteren Ausführungsform dient diese haarkosmetische oder haut- oder zahnkosmetische Zubereitung der Pflege oder dem Schutz der Haut oder Haars oder Zähne und liegt in Form einer Emulsion, einer Dispersion, einer Suspension, einer wässrigen Tensidzubereitung, einer Milch, einer Lotion, einer Creme, eines Balsams, einer Salbe, eines Gels, eines Granulats, eines Puders, eines Stiftpräparates, wie z.B. eines Lippenstifts, eines Schaums, eines Aerosols oder eines Sprays vor. Solche Formulierungen sind gut geeignet für topische Zubereitungen. Als Emulsionen kommen ÖI-in-Wasser-Emulsionen (O/W-Typ) und Wasser-in-Öl-Emulsionen (W/O-Typ) oder Mikroemulsionen in Frage.

Im Regelfall wird die haarkosmetische, zahnkosmetische oder hautkosmetische Zubereitung zur Applikation auf der Haut (topisch), Zähne oder Haar verwendet. Unter topischen Zubereitungen sind dabei solche Zubereitungen zu verstehen, die dazu geeignet sind, die Wirkstoffe in feiner Verteilung und bevorzugt in einer durch die Haut resorbierbaren Form auf die Haut aufzubringen. Hierfür eignen sich z.B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer bevorzugten Ausführungsform des erfindungsgemäßen kosmetischen Mittels enthält das Mittel einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Als Emulgatoren können nichtionogene Tenside, zwitterionische Tenside, ampholytische Tenside oder anionische Emulgatoren verwendet werden. Die Emulgatoren können in der erfindungsgemäßen Zusammensetzung in Mengen von 0,1 bis 10, vorzugsweise 1 bis 5 Gew.-%, bezogen auf die Zusammensetzung, enthalten sein.

Als nichtionogenes Tensid kann beispielsweise ein Tensid aus mindestens einer der folgenden Gruppen verwendet werden:
Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
C_{12/18}-Fettsäuremono- und -diester von Anlagerungsprodukten von 1 bis 30 Mol Ethylenoxid an Glycerin;
Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
Alkylmono- und -oligoglycoside mit 8 bis 22 Kohlenstoffatomen im Alkylrest und deren ethoxylierte Analoga;
Anlagerungsprodukte von 15 bis 60 Mol Ethylenoxid an Ricinusöl und/oder gehärtetes Ricinusöl;
Polyol- und insbesondere Polyglycerinester, wie z.B. Polyglycerinpolyricinoleat, Polygl ycerinpoly-12-hydroxystearat oder Polyglycerindimerat. Ebenfalls geeignet sind Gemische von Verbindungen aus mehreren dieser Substanzklassen; Anlagerungsprodukte von 2 bis 15 Mol Ethylenoxid an Ricinusöl und/oder gehärtetes Ricinusöl;
Partialester auf Basis linearer, verzweigter, ungesättigter bzw. gesättigter *C*_{6/22} *-* Fettsäuren, Ricinolsäure sowie 12-Hydroxystearinsäure und Glycerin, Polyglycerin, Pentaerythrit, Dipenta-erythrit, Zuckeralkohole (z. B. Sorbit), Alkylglucoside (z.B. Methylglucosid, Butylglucosid, Lauryl-glucosid) sowie Polyglucoside (z.B. Cellulose);
Mono-, Di- und Trialkylphosphate sowie Mono-, Di- und/oder Tri-PEG-alkylphosphate und deren Salze;
Wollwachsalkohole;
Polysiloxan-Polyalkyl-Polyether-Copolymere bzw. entsprechende Derivate;
Mischester aus Pentaerythrit, Fettsäuren, Citronensäure und Fettalkohol gemäß DE PS 1165574 und/oder Mischester von Fettsäuren mit 6 bis 22 Kohlenstoffatomen, Methylglucose und Polyolen, vorzugsweise Glycerin oder Polyglycerin sowie Polyalkylenglycole;
Betaine.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethyl-carboxymethylglycinat. Besonders bevorzugt ist das unter der CTFA Bezeichnung Cocamidopropyl Betaine bekannte Fettsäureamid-Derivat.

Ebenfalls geeignete Emulgatoren sind ampholytische Tenside. Unter ampholytischen Tensiden werden solche oberflächenaktiven Verbindungen verstanden, die außer einer C_{8,18}-Alkyl- oder -Acylgruppe im Molekül mindestens eine freie Aminogruppe und mindestens eine -COOH- oder -S0₃H-Gruppe enthalten und zur Ausbildung innerer Salze befähigt sind. Beispiele für geeignete ampholytische Tenside sind N-Alkylglycine, N-Alkylpropionsäuren , N-Alkylamino-buttersäuren , N Alkyliminodipropionsäuren, N-Hydroxyethyl-N-alkylamido-propylglycine, N-Alkyltaurine, N Alkylsarcosine, 2-Alkylaminopropionsäuren und Alkylaminoessigsäuren mit jeweils etwa 8 bis 18 C-Atomen in der Alkylgruppe.

Besonders bevorzugte ampholytische Tenside sind das N-Kokosalkyl-aminopropionat, Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden.

Als Ölkörper kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettaikohoien, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-, Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv 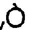 TN), Dialkylether, Ringöffnungsprodukte von epoxidierten Fettsäureestern mit Polyolen, Siliconöle und/oder aliphatische bzw. naphthenische Kohlenwasserstoffe in Betracht. Als Ölkörper können ferner auch Siliconverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80, und insbesondere 10 bis 50 Gew.-%, bezogen auf die Zusammensetzung enthalten sein. Insbesondere für Deodorantien sind geeignete Effektormoleküle (ii) : Parfumöle, Cyclodextrine, Ionenaustauscher, Zink-Ricinoleat, keimhemmende/bakteriostatische Verbindungen (z.B. DCMX, Irgasan DP 300, TCC).

Für Antitranspirantien geeignet sind: Tannine, und Zink-/Aluminiumsalze.

Die Zubereitungen enthalten neben den beschriebenen Hilfsstoffen Hydrophobin in einem Anteil ausgewählt aus der Gruppe bestehend aus 0,000001 bis 10 Gew.-%, 0,0001 bis 10 Gew.-%, 0,001 bis 10 Gew.-%, 0,01 bis 10 Gew.-%, 0,1 bis 10 Gew.-% und 1 bis 10 Gew.-% ,

In einer Ausführung der vorliegenden Erfindung wird Hydrophobin als Penetrationsverstärker in Pflanzenschutzmitteln verwendet.

Gegenstand der vorliegenden Erfindung istferner ein Verfahren zur Herstellung von Pflanzenschutzmitteln enthaltend Hydrophobin sowie Pflanzenschutzmitteln enthaltend Hydrophobin.

Die Pflanzenschutzmittel enthalten neben den beschriebenen Hilfsstoffen Hydrophobin in einem Anteil ausgewählt aus der Gruppe bestehend aus 0,000001 bis 10 Gew.-%, 0,0001 bis 10 Gew.%, 0,001 bis 10 Gew.%, 0,01 bis 10 Gew.%, 0,1 bis 10 Gew.-% und 1 bis 10 Gew.-%,

Der Gehalt an Wirk- und/oder Effektstoff kann über weite Bereiche variiert werden. Insbesondere ermögüchen die amphiphilen Polymerzusammensetzungen das Herstellen von so genannten Wirkstoffkonzentraten, welche den Wirkstoff in einer Menge von wenigstens 5 Gew.%, z. B. in einer Menge von 5 bis 50 Gew.% und insbesondere in einer Menge von 5 bis 20 Gew.-%, bezogen auf das Gesamtgewicht der Zusammensetzung, enthalten.

Vorteilhafterweise können die erfindungsgemäßen wäßrigen Wirkstoffzusammensetzungen lösungsmittelfrei oder lösungsmittelarm formuliert werden, d. h. der Anteil an organischen Lösemitteln in der wäßrigen Wirkstoffzusammensetzung beträgt häufig nicht mehr als 10 Gew.%, insbesondere nicht mehr als 5 Gew.% und insbesondere nicht mehr als 1 Gew.-%, bezogen auf das Gesamtgewicht der Zusammensetzung.

In den erfindungsgemäßen wäßrigen Zusammensetzungen können eine Vielzahl unterschiedlicher Wirk- und Effektstoffe formuliert werden. Eine besondere Ausführungsform der Erfindung betrifft die Formulierung von Wirkstoffen für den Pflanzenschutz, d.h. von Herbiziden, Fungiziden, Nematiziden, Akariziden, Insektiziden sowie Wirkstoffen, die das Pflanzenwachstum regulieren.

Beispiele für fungizide Wirkstoffe, die als erfindungsgemäße wässrige Wirkstoffzusammensetzung formuliert werden können, umfassen:
- Acylalanine wie Benalaxyl, Metalaxyl, Ofurace, Oxadixyl;
- Aminderivate wie Aldimorph, Dodine, Dodemorph, Fenpropimorph, Fenpropidin, Guazatine, Iminoctadine, Spiroxamin, Tridemorph;
- Anilinopyrimidine wie Pyrimethanil, Mepanipyrim oder Cyrodinyl;
- Antibiotika wie Cycloheximid, Griseofulvin, Kasugamycin, Natamycin, Polyoxin und Streptomycin;
- Azole wie Bitertanol, Bromoconazol, Cyproconazole, Difenoconazol, Dinitroconazol, Epoxiconazol, Fenbuconazol, Fluquiconazol, Flusilazol, Flutriafol, Hexaconazol, Imazalil, Ipconazol, Metconazol, Myclobutanil, Penconazol, Propiconazol, Prochloraz, Prothioconazol, Tebuconazol, Tetraconazol, Triadimefon, Triadimenol, Triflumizol, Triticonazol;
- 2-Methoxybenzophenone, wie sie in EP-A 897904 durch die allgemeine Formel I beschrieben werden, z.B. Metrafenon;
- Dicarboximide wie Iprodion, Myclozolin, Procymidon, Vinclozolin;
- Dithiocarbamate wie Ferbam, Nabam, Maneb, Mancozeb, Metam, Metiram, Propineb, Polycarbamat, Thiram, Ziram, Zineb;
- Heterocyclische Verbindungen wie Anilazine, Benomyl, Boscalid, Carbendazim, Carboxin, Oxycarboxin, Cyazofamid, Dazomet, Dithianon, Famoxadon, Fenamidon, Fenarimol, Fuberidazole, Flutolanil, Furametpyr, Isoprothiolane, Mepronil, Nuarimol, Picobezamid, Probenazole, Proquinazid, Pyrifenox, Pyroquilon, Quinoxyfen, Silthiofam; Thiabendazole, Thifluzamid, Thiophanat-methyl, Tiadinil, Tricyclazole, Triforine;
- Nitrophenylderivative wie Binapacryl, Dinocap, Dinobuton, Nitrophthal-isopropyl;
- Phenylpyrrole wie Fenpiclonil sowie Fludioxonil;
- nicht klassifizierte Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminum, Iprovalicarb, Hexachlorobenzol, Metrafenon, Pencycuron, Propamocarb, Phthalide, Toloclofos-Methyl, Quintozene, Zoxamid;
- Strobilurine wie sie in WO 03/075663 durch die allgemeine Formel I beschrieben werden, beispielsweise Azoxystrobin, Dimoxystrobin, Fluoxastrobin, Kresoxim-methyl, Metominostrobin, Orysastrobin, Picoxystrobin, Pyraclostrobin sowie Trifloxystrobin;
- Sulfensäure-Derivative wie Captafol, Captan, Dichlofluanid, Folpet, Tolylfluanid;
- Zimtsäureamide und Analoga wie Dimethomorph, Flumetover, Flumorp;
- 6-Aryl-[1,2,4]triazolo[1,5-a]pyrimidine wie sie z.B. in WO 98/46608, WO 99,41255. oder WO 03/004465 (jeweils durch die allgemeine Formel I beschrieben werden (Seite 1, Zeile 8 bis Seite 11, Zeile 45, sowie Verbindungen dargestellt in Formel IA in Verbindung mit Tabellen 1 bis 44 und Tabelle Ader WO 03/00465);
- Amidfungizide wie Cyclofenamid sowie (Z)-N-[α-(Cyclopropylmethoxyimino)-2,3-difluoro-6-(difluoromethoxy)benzyl]-2-phenylacetamid.

### Beispiele für Herbizide, die als erfindungsgemäße wässrige Wirkstoffzusammensetzung formuliert werden können, umfassen:

- 1,3,4-Thiadiazole wie Buthidazole und Cyprazole;
- Amide wie Allidochlor, Benzoylpropethyl, Bromobutide, Chlorthiamid, Dimepiperate, Dimethenamid, Diphenamid, Etobenzanid, Flampropmethyl, Fosamin, Isoxaben, Metazachlor, Alachlor, Acetochlor, Metolachlor, Monalide, Naptalame, Pronamid, Propanil;

- Aminophosphorsäuren wie Bilanafos, Buminafos, Glufosinateammonium, Glyphosate, Sulfosate;
- Aminotriazole wie Amitrol, Anilide wie Anilofos, Mefenacet;
- Aryloxyalkan-säure wie 2,4-D, 2,4-DB, Clomeprop, Dichlorprop, Dichlorprop-P, Dichlorprop-P, Fenoprop, Fluroxypyr, MCPA, MCPB, Mecoprop, Mecoprop-P, Napropamide, Napro-panilide, Triclopyr;
- Benzoesäuren wie Chloramben, Dicamba;
- Benzothiadiazinone wie Bentazon;
- Bleacher wie Clomazone, Diflufenican, Fluorochloridone, Flupoxam, Fluridone, Pyrazolate, Sulcotrione;
- Carbamate wie Carbetamid, Chlorbufam, Chlorpro-pham, Desmedipham, Phenmedipham, Vernolate;
- Chinolinsäuren wie Quinclorac, Quinmerac;
- Dichlorpropionsäuren wie Dalapon;
- Dihydrobenzofurane wie Ethofumesate;
- Dihydrofuran-3-on wie Flurtamone;
- Dinitroaniline wie Benefin, Butralin, Dinitramin, Ethalfluralin, Fluchloralin, Isopropalin, Nitralin, Oryzalin, Pendimethalin, Prodiamine, Profluralin, Trifluralin, Dinitrophenole wie Bromofenoxim, Dinoseb, Dinoseb-acetat, Dinoterb, DNOC, Minoterb-Acetat;
- Diphenylether wie Acifluorfen-sodium, Aclonifen, Bifenox, Chlornitrofen, Difenoxuron, Ethoxyfen, Fluorodifen, Fluoroglycofen-ethyl, Fomesafen, Furyloxyfen, Lactofen, Nitrofen, Nitrofluorfen, Oxyfluorfen;
- Dipyridyle wie Cyperquat, Difenzoquat-methylsulfat, Diquat, Paraquat-dichlorid;
- Imidazole wie Isocarbamid;
- Imidazolinone wie Imazamethapyr, Imazapyr, Imazaquin, Imazethabenz-methyl, Imazethapyr, Imazapic, Imazamox;
- Oxadiazole wie Methazole, Oxadiargyl, Oxadiazon;
- Oxirane wie Tridiphane;
- Phenole wie Bromoxynil, loxynil;
- Phenoxyphenoxypropionsäureester wie Clodinafop, Cyhalofop-butyl, Diclofopmethyl, Fenoxaprop-ethyl, Fenoxaprop-p-ethyl, Fenthiapropethyl, Fluazifop-butyl, Fluazifop-p-butyl, Haloxyfop-ethoxy-ethyl, Haloxyfop-methyl, Haloxyfop-p-methyl, Isoxapyrifop, Propaquizafop, Quizalofop-ethyl, Quizalofop-p-ethyl, Quizalofop-tefuryl;
- Phenylessigsäuren wie Chlorfenac;
- Phenylpropionsäuren wie Chlorophenprop-methyl;
- ppi-Wirkstoffe wie Benzofenap, Cinidon-Ethyl, Flumiclorac-pentyl, Flumioxazin, Flumipropyn, Flupropacil, Pyrazoxyfen, Sulfentrazone, Thidiazimin;
- Pyrazole wie Nipyraclofen;
- Pyridazine wie Chloridazon, Maleic hydrazide, Norflurazon, Pyridate;
- Pyridincarbonsäuren wie Clopyralid, Dithiopyr, Picloram, Thiazopyr;
- Pyrimidylether wie Pyrithiobacsäure, Pyrithiobac-sodium, KIH-2023, KIH-6127;
- Sulfonamide wie Flumetsulam, Metosulam;
- Triazolcarboxamide wie Triazofenamid;
- Uracile wie Bromacil, Lenacil, Terbacil;
- ferner Benazolin, Benfuresate, Bensulide, Benzofluor, Bentazon, Butamifos, Cafenstrole, Chlorthal-dimethyl, Cinmethylin, Dichlobenil, Endothall, Fluorbentranil, Mefluidide, Perfluidone, Piperophos, Topramezone und Prohexandion-Calcium;
- Sulfonylharnstoffe wie Amidosulfuron, Azimsulfuron, Bensulfuron-methyl, Chlorimuron-ethyl, Chlorsulfuron, Cinosulfuron, Cyclosulfamuron, Ethametsulfuronmethyl, Flazasulfuron, Halosulfuron-methyl, Imazosulfuron, Metsulfuron-methyl, Nicosulfuron, Primisulfuron, Prosulfuron, Pyrazosulfuron-ethyl, Rimsulfuron, Sulfometuron-methyl, Thifensulfuron-methyl, Triasulfuron, Tribenuron-methyl, Triflusulfuron-methyl, Tritosulfuron;
- Pflanzenschutz-Wirkstoffe vom Cyclohexenon-Typ wie Alloxydim, Clethodim, Cloproxydim, Cycloxydim, Sethoxydim und Tralkoxydim. Ganz besonders bevorzugte herbizide Wirkstoffe vom Cyclohexenon-Typ sind: Tepraloxydim (vgl. AGROW, Nr. 243, 3.11.95, Seite 21, Caloxydim) und 2-(1-[2-{4-Chlorphenoxy}propyl-oxyimino]butyl)-3-hydroxy-5-(2H-tetrahydrothiopyran-3-yl)-2-cyclohexen-1-on und vom Sulfonylharnstoff-Typ: N-(((4-methoxy-6-[trifluormethyl]-1,3,5-triazin-2-yl)amino)carbo-nyl)-2-(trifluormethyl)-benzolsulfonamid.

### Beispiele für Insektizide, die als erfindungsgemäße wässrige

Wirkstoffzusammensetzung formuliert werden können, umfassen:
- Organophosphate wie Acephate, Azinphos-methyl, Chlorpyrifos, Chlorfenvinphos, Diazinon, Dichlorvos, dimethylvinphos, dioxabenzofos, Dicrotophos, Dimethoate, Disulfoton, Ethion, EPN, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Primiphos-ethyl, Pyraclofos, Pyridaphenthion, Sulprophos, Triazophos, Trichlorfon; Tetrachlorvinphos, Vamidothion

- Carbamate wie Alanycarb, Benfuracarb, Bendiocarb, Carbaryl, carbofuran, Carbosulfan, Fenoxycarb, Furathiocarb, Indoxacarb, Methiocarb, Methomyl, Oxamyl, Pirimicarb, Propoxur, Thiodicarb, Triazamate;
- Pyrethroide wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin, Permethrin;
- Arthropode Wachstumsregulatoren: a) Chitinsyntheseinhibitoren z.B. Benzoylharnstoffe wie Chlorfluazuron, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) Ecdysone Antagonisten wie Halofenozide, Methoxyfenozide, Tebufenozide; c) Juvenoide wie Pyriproxyfen, Methoprene, Fenoxycarb; d) Lipid-Biosyntheseinhibitoren wie Spirodiclofen;
- Neonicotinoide wie Flonicamid, Clothianidin, Dinotefuran, Imidacloprid, Thiamethoxam, Nitenpyram, Nithiazin, Acetamiprid, Thiacloprid;
- Weitere unklassifizierte Insektizide wie Abamectin, Acequinocyl, acetamiprid, Amitraz, Azadirachtin, Bensultap Bifenazate, Cartap, Chlorfenapyr, Chlordimeform, Cyromazine, Diafenthiuron, Dinetofuran, Diofenolan, Emamectin, Endosulfan, Ethiprole, Fenazaquin, Fipronil, Formetanate, Formetanate hydrochlorid, gamma-HCH Hydramethylnon, Imidacloprid, Indoxacarb, Isoprocarb, Metolcarb, Pyridaben, Pymetrozine, Spinosad, Tebufenpyrad, Thiamethoxam, Thiocyclam, XMC und Xylylcarb und
- N-Phenylsemicarbazone, wie sie in EP-A 462 456 durch die allgemeine Formel I beschrieben werden, insbesondere Verbindungen der allgemeinen Formel IV 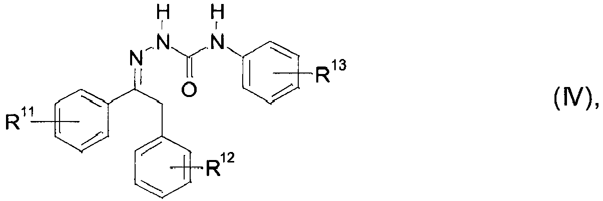
worin R11 und R12 unabhängig voneinander für Wasserstoff, Halogen, CN, C1-C4-Alkyl, C1-C4-Alkoxy, C1-C4-Haloalkyl oder C1-C4-Haloalkoxy stehen und R13 für C1-C4-Alkoxy, C1-C4-Haloalkyl oder C1-C4-Haloalkoxy steht, z.B. Verbindung IV, worin R1 für 3-CF3 und R2 für 4-CN stehen und R3 4-OCF3 bedeutet.

Brauchbare Wachstumsregulatoren sind z.B. Chlormequat-chlorid, Mepiquatchlorid, Prohexadion-Calcium oder die die Gruppe der Gibberelline. Dazu gehören z.B. die Gibberelline GA1, GA3, GA4, GA5 und GA7 etc. und die entsprechenden exo-16,17-Dihydrogibberelline sowie die Derivate davon, z.B. die Ester mit C1-C4-Carbonsäuren. Erfindungsgemäß bevorzugt ist das exo-16,17-Dihydro-GA5-13-acetat.

Eine bevorzugte Ausführungsform der Erfindung betrifft die erfindungsgemäße Verwendung von Hydrophobin zur Herstellung wässriger Wirkstoffzusammensetzungen von Fungiziden, insbesondere Strobilurinen, Azolen und 6-Aryltriazolo (1,5a] pyrimidinen, wie sie z.B. in WO 98/46608, WO 99/41255 oder WO 03/004465 jeweils durch die allgemeine Formel beschrieben werden (Seite 1, Zeile 8 bis Seite 11, Zeile 45, sowie Verbindungen dargestellt in Formel IA in Verbindung mit Tabellen 1 bis 44 und Tabelle Ader WO 03/00465), insbesondere für Wirkstoffe der allgemeinen Formel V, 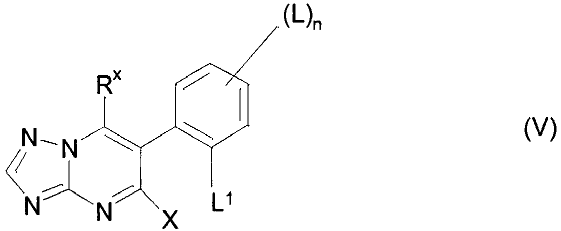 worin:
Rx für eine Gruppe NR14R15 steht, oder lineares oder verzweigtes C1-C8-Alkyl, das gegebenenfalls durch Halogen, OH, C1-C4-Alkoxy, Phenyl oder C3-C6-Cycloalkyl substiuiert ist, C2-C6-Alkenyl, C3-C6-Cycloalkyl, C3-C6-Cycloalkenyl, Phenyl oder
Naphthyl, wobei die 4 zuletzt genannten Reste 1, 2, 3 oder 4 Substituenten ausgewählt unter Halogen, OH, C1-C4-Alkyl, C1-C4-Halogenalkoxy, C1-C4-Alkoxy und C1-C4-Halogenalkyl aufweisen können;
R14, R15 unabhängig voneinander für Wasserstoff, C1-C8-Alkyl, C1-C8-Halogenalkyl, C3-C10-Cycloalkyl, C3-C6-Halogencycloalkyl, C2-C8-Alkenyl, C4-C10-Alkadienyl, C2-C8-Halogenalkenyl, C3-C6-Cycloalkenyl, C2-C8-Halogencycloalkenyl, C2-C8-Alkinyl, C2-C8-Halogenalkinyl oder C3-C6-Cycloalkinyl,
R14 und R15 zusammen mit dem Stickstoffatom, an das sie gebunden sind, fünf- bis achtgliedriges Heterocyclyl, welches über N gebunden ist und ein, zwei oder drei weitere Heteroatome aus der Gruppe O, N und S als Ringglied enthalten und/oder einen oder mehrere Substituenten aus der Gruppe Halogen, C1-C6-Alkyl, C1-C6-Halogenalkyl, C2-C6-Alkenyl, C2-C6-Halogenalkenyl, C1-C6-Alkoxy, C1-C6-Halogenalkoxy, C3-C6-Alkenyloxy, C3-C6-Halogenalkenyloxy, (exo)-C1-C6-Alkylen und Oxy-C1-C3-alkylenoxy tragen kann;
L ausgewählt ist unter Halogen, Cyano, C1-C6-Alkyl, C1-C4-Halgoenalkyl, C1-C6-Alkoxy, C1-C4-Halgoenalkoxy und C1-C6-Alkoxycarbonyl;
L1 Halogen, C1-C6-Alkyl oder C1-C6-Halogenalkyl und insbesondere Fluor oder Chlor bedeutet;
X für Halogen, C1-C4-Alkyl, Cyano, C1-C4-Alkoxy oder C1-C4-Halogenalkyl und vorzugsweise für Halogen oder Methyl steht und insbesondere Chlor bedeutet.

### Beispiele für Verbindungen der Formel V sind

5-Chlor-7-(4-methylpiperidin-1-yl)-6-(2,4,6-trifluorphenyl)-[1,2,4]triazolo[1,5-a]pyrimidin,
5-Chlor-7-(4-methylpiperazin-1-yl)-6-(2,4,6-trifluorphenyl)-[1,2,4]triazolo[1,5-a]pyrimidin,
5-Chlor-7-(morpholin-1-yl)-6-(2,4,6-trifluorphenyl)-[1,2,4jtriazolo[1,5-a]pyrimidin,
5-Chlor-7-(piperidin-1 -yl)-6-(2,4,6-trifluorphenyl)-[1,2,4]triazolo[1,5-a]pyrimidin,
5-Chlor-7-(morpholin-1-yl)-6-(2,4,6-trifluorphenyl)-[1,2,4]triazolo[1,5-a]pyrimidin,
5-Chlor-7-(isopropylamino)-6-(2,4,6-trifluorphenyl)-[1,2,4]triazolo[1,5-a]pyrimidin,
5-Chlor-7-(cyclopentylamino)-6-(2,4,6-trifluorphenyl)-[1,2,4]triazolo[1,5-a]pyrimidin,
5-Chlor-7-(2,2,2-trifluorethylamino)-6-(2,4,6-trifluorphenyl)-[1,2,4]triazoto[1,5-a]pyrimidin,
5-Chlor-7-(1,1,1-trifluorpropan-2-ylamino)-6-(2,4,6-trifluorphenyl)-[1,2,4]triazolo[1,5-a]pyrimidin,
5-Chlor-7-(3,3-dimethylbutan-2-ylamino)-6-(2,4,6-trifluorphenyl)-[1,2,4]triazolo[1,5-a]pyrimidin,
5-Chlor-7-(cyclohexylmethyl)-6-(2,4,6-trifluorphenyl)-[1,2,4]triazolo[1,5-a]pyrimidin,
5-Chlor-7-(cyclohexyl)-6-(2,4,6-trifluorphenyl)-(1,2,4]triazolo[1,5-a]pyrimidin,
5-Chlor-7-(2-methylbutan-3-yl)-6-(2,4,6-trifluorphenyl)-[1,2,4]triazolo[1,5-a]pyrimidin,
5-Chlor-7-(3-methylpropan-1-yl)-6-(2,4,6-trifluorphenyl)-[1,2,4]triazolo[1,5-a]pyrimidin,
5-Chlor-7-(4-methylcyclohexan-1-yl)-6-(2,4,6-trifluorphenyl)-[1,2,4]triazolo[1,5-a]pyrimidin,
5-Chlor-7-(hexan-3-yl)-6-(2,4,6-trifluorphenyl)-[1,2,4]triazolo[1,5-a]pyrimidin,
5-Chlor-7-(2-methylbutan-1-yl)-6-(2,4,6-trifluorphenyl)-(1,2,4]triazolo[1,5-a]pyrimidin,
5-Chlor-7-(3-methylbutan-1-yl)-6-(2,4,6-trifluorphenyl)-(1,2,4]triazolo[1,5-a]pyrimidin,
5-Chlor-7-(1-methylpropan-1-yl)-6-(2,4,6-trifluorphenyl)-[1,2,4]triazolo[1,5-a]pyrimidin,
5-Methyl-7-(4-methylpiperidin-1-yl)-6-(2,4,6-trifluorphenyl)-[1,2,4]triazolo[1,5-a]pyrimidin,
5-Methyl-7-(4-methylpiperazin-1-yl)-6-(2,4,6-trifluorphenyl)-[1,2,4]triazolo[1,5-a]pyrimidin,
5-Methyl-7-(morpholin-1-yl)-6-(2,4,6-trifluorphenyl)-[1,2,4]triazolo[1,5-a]pyrimidin,
5-Methyl-7-(piperidin-1-yl)-6-(2,4,6-trifluorphenyl)-[1,2,4]triazolo[1,5-a]pyrimidin,
5-Methyl-7-(morpholin-1-yl)-6-(2,4,6-trifluorphenyl)-[1,2,4]triazolo[1,5-a]pyrimidin,
5-Methyl-7-(isopropylamino)-6-(2,4,6-trifluorphenyl)-[1,2,4]triazolo[1,5-a]pyrimidin,
5-Methyl-7-(cyclopentylamino)-6-(2,4,6-trifluorphenyl)-[1,2,4]triazolo[1,5-a]pyrimidin,
5-Methyl-7-(2,2,2-trifluorethylamino)-6-(2,4,6-trifluorphenyl)-[1,2,4]triazolo[1,5-a]pyrimidin,
5-Methyl-7-(1,1,1-trifluorpropan-2-ylamino)-6-(2,4,6-trifluorphenyl)-[1,2,4]triazolo[1,5-a]pyrimidin,
5-Methyl-7-(3,3-dimethylbutan-2-ylamino)-6-(2,4,6-trifluorphenyl)-[1,2,4]triazolo[1,5-a]pyrimidin,
5-Methyl-7-(cyclohexylmethyl)-6-(2,4,6-trifluorphenyl)-[1,2,4]triazolo[1,5-a]pyrimidin,
5-Methyl-7-(cyclohexyl)-6-(2,4,6-trifluorphenyl)-[1,2,4]triazolo[1,5-a]pyrimidin,
5-Methyl-7-(2-methylbutan-3-yl)-6-(2,4,6-trifluorphenyl)-[1,2,4]triazolo[1,5-a]pyrimidin,
5-Methyl-7-(3-methylpropan-1-yl)-6-(2,4,6-trifluorphenyl)-[1,2,4]triazolo[1,5-a]pyrimidin,
5-Methyl-7-(4-methylcyclohexan-1-yl)-6-(2,4,6-trifluorphenyl)-[1,2,4]triazolo[1,5-a]pyrimidin,
5-Methyl-7-(hexan-3-yl)-6-(2,4,6-trifluorphenyl)-[1,2,4]triazolo[1,5-a]pyrimidin,
5-Methyl-7-(2-methylbutan-1-yl)-6-(2,4,6-trifluorphenyl)-[1,2,4]triazolo[1,5-a]pyrimidin,
5-Methyl-7-(3-methylbutan-1-yl)-6-(2,4,6-trifluorphenyl)-[1,2,4]triazolo[1,5-a]pyrimidin
und 5-Methyl-7-(1-methylpropan-1-yl)-6-(2,4,6-trifluorphenyl)-[1,2,4]triazolo[1,5-a]pyrimidin.

Eine weitere bevorzugte Ausführungsform der Erfindung betrifft die Verwendung von Hydrophobin als Penetrationsverstärker zur Herstellung wässriger Wirkstoffzusammensetzungen von Insektiziden, insbesondere von Arylpyrrolen wie Chlorfenapyr, von Pyrethroiden wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin und Permethrin, von Neonicotinoiden und von Semicarbazonen der Formel IV, von Fipronil.

In einer Ausführung der vorliegenden Erfindung führt die Verwendung von Hydrophobin als Penetrationsverstärker zu einer Herabsetzung der für die gewünschte zu erzielende Wirkung notwendigen Konzentration an Wirkstoffen um 1%, 2%, 3%, 4%, 5%, %, 7%, 8%, 9%, 10%, bevorzugt 11%, 12%,13%, 14%, 15%,16%,18%, 20%, besonders bevorzugt 22%, 25% 30%, 35%, 40%, 45%, 50%, insbesondere 60%, 70%, 80%, 90%.

In einer Ausführung der vorliegenden Erfindung wird zunächst eine phosphatgepufferte Lösung auf die zu behandelnde Oberfläche, bzw. Phasengrenze aufgetragen. Hydrophobin in einer Konzentration von 0,01 bis 0,2 Gewichtsprozent wird in 50mM NaH2PO4 mit pH 7,5 gelöst.

Nach Inkubation mit der Hydrophobin-Lösung erfolgt das Auftragen der Zubereitung enthaltend mindestens einen Wirkstoff.

Weiterer Gegenstand der vorliegende Erfindung ist ein Verfahren zur verbesserten Aufnahme von Wirkstoffen bei topischer Anwendung dadurch gekennzeichnet, daß Hydrophobin
a) vor den Wirkstoffen
   oder
b) gleichzeitig mit den Wirkstoffen appliziert wird.

Weiterer Gegenstand der vorliegende Erfindung ist ein Verfahren zur Herstellung eines Mittels zur verbesserten Aufnahme von Wirkstoffen bei topischer Anwendung dadurch gekennzeichnet, daß Hydrophobin in fester Form, in Lösung oder in Dispersion in einem organischen oder in einem anorganischen Medium in eine Zubereitung enthalten mindestens einen Wirkstoff eingebracht wird.

### Beispiele:

### Beispiel 1 a:

Hintergrund ist die Überlegung, dass die Inkubation der Zellen mit den antioxidativ wirksamen Referenzsubstanzen, unter Einfluss von Hydrophobin (Hydropobin A (SEQ ID 20 aus WO2007/14897) oder B (SEQ ID 26 aus W02007/14897))
als Penetrationsverstärker zu einem verstärkten antioxidativen Potential der führt. Als Kontrollen wurden neben den Kulturen, welche mit Referenzsubstanzen und ohne Penetrationsverstärker kultiviert wurden, unbehandelte Kulturen und vehikelbehandelte Kulturen eingesetzt.

Zur Dosisfindung wurden, die einsetzbaren Konzentrationen vor Beginn der Hauptversuche mittels eines Zytotoxizitätsassays (hier durch MTT Umsatz) geprüft. Aufgrund dieser Vorversuche wurde für die Bestimmung des Einfusses der Proteine auf die antioxidative Kapazität von Vitamin C, Tocopherylacetat und Quercetin sowie im Hinblick auf eine ökonomische Verwendung, eine Konzentration von 0,001 % gewählt.

Als Referenzsubstanzen wurden Vitamin E (alpha-Tocopherylacetat), Vitamin C (Mg-Ascorbylphosphat) und Quercetin eingesetzt. Als Testzellen wurden normale dermale Bindegewebszellen (Fibroblasten) eingesetzt, da diese im Evaluationsverfahren eine gute Signalstärken erbrachten.

Zur Ermittlung der antioxidativen Eigenschaften der Lösungen wurden die NHDFKulturen (normale humane dermale Fibroblasten) auf 48-Well-Kuturgefäße ausgesät und bis zur vollständigen Bedeckung der Kulturoberfläche kultiviert. Mit diesen statischen Kulturen wurden anschließend die Untersuchungen durchgeführt. Zunächst wurden die Kulturen für 24 h mit den Testlösungen behandelt. Anschließend wurde das Medium (incl. Testlösungen) abgenommen, die Kulturen wurden mit Puffer gewaschen und mit dem Fluoreszenzfarbstoff (DCFH) inkubiert. Anschließend wurde zur Entfernung nicht aufgenommenen Farbstoffes mehrmals gewaschen und die Zellen mit dem farblosen Assaymedium versetzt. Die Platten mit den Zellen wurden in einen Fluoreszenzreader eingesetzt und die Messung mit einer einleitenden Phase ohne Stress gestartet. Durch die Zugabe von H₂O₂ wurden anschließend mehrfach intrazelluläre, freie Radikale induziert, die mit dem Farbstoff einem fluoreszierenden Derivat reagieren. Werden die freien Radikale allerdings zuvor von Antioxidantien gequencht wird die Entstehung fluoreszierender Derivate verhindert bzw. vermindert. Geringe Fluoreszenz zeigt also hohe antioxidative Kapazität an. Diese Versuche wurden mit jeweils 6 Replikaten in drei unabhängigen Durchgängen durchgeführt.

Im Anschluss werden die Ergebnisse der Untersuchungen als Graphiken dargestellt. Aus der gesamten Laufzeit von 135 min wurde, aus Zwecken der Übersichtlichkeit jeweils nach 90 min eine Momentaufnahme erstellt (die vollständigen Darstellungen werden im Anhang gezeigt). Die γ-Achse stellt dabei den oxidativen Stress in den Zellen als die Fluoreszenz dar, welche emmitiert wird wenn freie Radikale mit dem intrazellulär vorliegenden Farbstoff DCFH reagieren. Dies bedeutet, dass ein niedriger Balken geringen oxidativen Stress und damit hohe antioxidative Kapazität der Zellen als Folge der Supplementierung symbolisiert. Alle Fluoreszenzwerte wurden mit den Proteingehalten der Kulturen nach Abschluss der Messungen korrigiert (ermittelt mit Coomassie-Färbung). Dadurch werden Schwankungen in der Zellzahl, welche sich auch in Schwankungen in der Fluoreszenz (aufgrund der unterschiedlichen Menge an Farbstoff) auswirken würden, ausgeglichen. Es werden also zellzahlbereinigte Daten dargestellt.

Tocopherylacetat alleine hat mit der eingesetzten Zelllinie in den durchgeführten Untersuchungen kein antioxidatives Potential.

Dennoch ist in zwei von drei Durchgängen ein verringerter oxidativer Stress bei den Kombinationen Hydrophobin A /Tocopherylacetat und Hydrophobin B / Tocopherylacetat zu beobachten. Dies ist kann als schwache positiver Effekt der Proteine interpretiert werden.

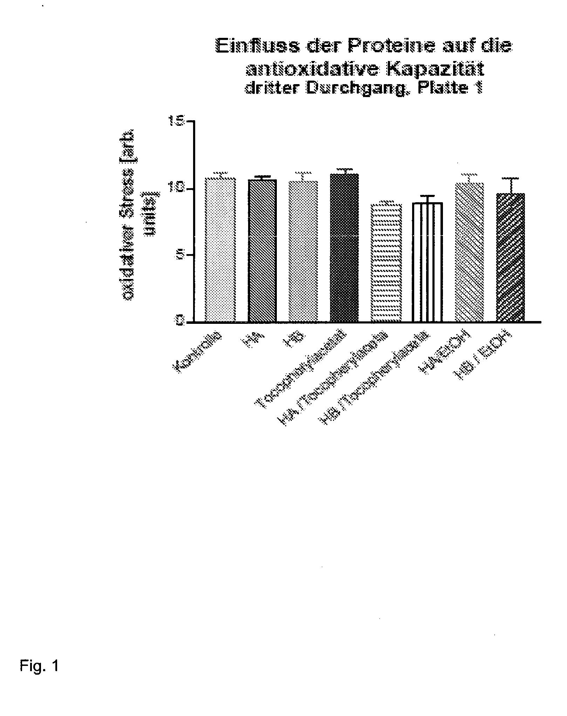

Die Hydrophobine alleine bedingen in zwei von drei Durchgängen einen gering erniedrigten oxidativen Stress welcher nur zum Teil für die Verringerung des oxidativen Stresses in den Kombinationen mit Tocopherylacetat verantwortlich sein kann. Im Dritten Durchgang wird dieser Effekt am deutlichsten. Die Proteine allein zeigen in diesem Durchgang keine Reduktion des oxidativen Stress während die Kombinationen aus den Proteinen und Tocopherylacetat einen Rückgang zeigen (Fig. 1).

### Beispiel 1 b:

Als weiterer Stoff mit antioxidativem Potential Quercitin wurde getestet, in seiner Wirkung auf die Reduktion von oxidativem Streß unter Versuchsbedingungen, Materialien und Methoden wir in Beispiel 1a).

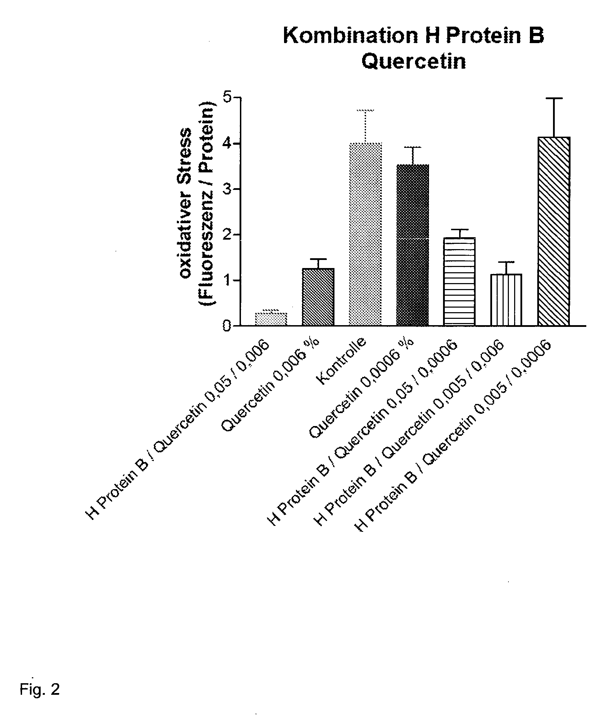

Die Ergebnisse (Fig. 2) zeigen, daß die Wirkung von Quercetin in Kombination mit Hydrophobin Protein B verbessert wurde (H Protein B 0,05 %, kombiniert mit Quercetin 0,0006 %).

### Beispiel 2:

Verbesserte Aufnahme und Bindung der Farbstoffe von Haartönungen durch Hydrophobin behandelte Haare.

Es wurden in diesem Test Euro-Naturhaare blond der Firma Kerling International Haarfabrik GmbH verwendet.

Bei den Haartönungen wurden handelsübliche Präparate (Stufe 1) getestet.

### Testvariation 1

Herstellen von Hydrophobin A oder B mit einer Konzentration von 0,01 bis 0,2 Gewichtsprozent. Lösungsmittel ist 50mM NaH2PO4 mit pH 7,5. Um die Lösung zu beschleunigen wird diese 1 h mittels Magnetrüher bei Raumtemperatur gelöst.

Zusätzlich wird eine Vergleichssuspension ohne Hydrophobin hergestellt.

Inkubation der Haare 1 Stunde bei 32°C mit anschließendem auswaschen mit Trinkwasser und trocknen.

Anwendung der Haartönung nach Herstellerangaben

Wiederholtes waschen (1 Minute einschäumen, 1 Minute ausspülen, trocknen bei Raumtemperatur) mit einer Penaten Baby-Shampoolösung (1%), und anschließender Beurteilung im getrockneten Zustand.

### Testvariation 2

Herstellen von Hydrophobin A oder B mit einer Konzentration von 0,01 bis 0,2 Gewichtsprozent. Lösungsmittel ist 50mM NaH2PO4 mit pH 7,5. Um die Lösung zu beschleunigen wird diese 1 h mittels Magnetrüher bei Raumtemperatur gelöst.

Zusätzlich wird eine Vergleichssuspension ohne Hydrophobin hergestellt.

Inkubation der Haare 1 Stunde bei 32°C mit anschließendem direkten trocknen bei 50°C mittels Fön.

Haare mit Trinkwasser auswaschen und bei Raumtemperatur trocknen.

Anwendung der Haartönung nach Herstellerangaben

Wiederholtes waschen (1 Minute einschäumen, 1 Minute ausspülen, trocknen bei Raumtemperatur) mit einer Penaten Baby-Shampoolösung (1%), und anschließender Beurteilung im getrockneten Zustand.

Besonders bei der ersten Testvariation konnte man bei den mit Hydrophobin A und B behandelten Haaren, eine verstärkte Bindung und Farbstoffaufnahme erkennen.

### Beispiel 3:

Verbesserte Haut-Penetration von Milchsäure durch eine Behandlung mit Hydrophobin Herstellen der Hydrophobinlösungen:
1. Herstellen einer 1%igen Lösung von Hydrophobin A und B in Milliporewasser Lösen des Hydrophobins durch kräftiges rühren auf einem Magnetrührer bei 900rpm für 45 Minuten.
2. Herstellen von 1 ml einer gebrauchsfertigen Hydrophobinlösung:
   ➢ 800ul Milliporewasser
   ➢ + 100µl 10-fach Puffer (500mM Tris, 10mM CaCl2, pH 8)
   ➢ + 100µl Hydrophobinlösung

Dies entspricht einer 0,1%igen Hydrophobinlösung in 50mM Tris 1mM CaCl2 und pH 8

Versuchsdurchführung:
1. Tiefgefrorene und enthaarte Schweinehaut (nicht gebrüht) wurde bei Raumtemperatur aufgetaut.
2. Einzeichnen der Applikationsfelder mit einer Größe von ca. 2cm x 2cm
3. Applizieren folgender Lösungen (stripping):
➢ Kontrolle (ohne Lösung)
➢ 100µl 0,1% H*A
➢ 100µl 0,1% H*B
4. Inkubation der Lösungen 1,5 Stunden bei RT
5. Um vorhandene Puffersalze zu entfernen, wurden alle Applikationsfelder mit 2x 500µl Milliporewasser abgespült und mit einem Papiertuch abgetupft.
6. Auftragen von jeweils 100µl 15%ige Milchsäure auf alle Applikationsfelder
7. Inkubation der Lösungen 10 Minuten bei Raumtemperatur
8. Entfernen der überschüssigen Milchsäure mit einem Papiertuch
9. Messung des pH-Wertes der Schweinehaut auf den jeweiligen Applikationsfeldern. Es wurde eine 3-fach Messung durchgeführt und den Mittelwert verwendet. Das nach einer pH-Messung vorhandene Wasser wurde mit einem Papiertuch abgetupft. Es wurde ein Skin-pH-Meter PH 905 der Firma Courage & Khazaka verwendet.
10. Abziehen der obersten losen Zellschicht mittels eines Corneofix - Klebestreifens der Firma Courage & Khazaka
11. Erneute Messung des pH-Wertes
12. Insgesamt wurde 3 mal ein Corneofix - Klebestreifen verwendet und danach der pH-Wert gemessen.

| pH-Werte | **Ohne** | **1x Stripping** | **2x Stripping** | **3x Stripping** |
|---|---|---|---|---|
| **Kontrolle** | 2,83 | 3,52 | 3,62 | 3,67 |
| **H*A** | 2,62 | 3,08 | 3,39 | 3,63 |
| **H*B** | 2,64 | 2,89 | 3,08 | 3,26 |

### Ergebnis:

Erkennbar ist, dass eine Beschichtung mit Hydrophobin, besonders mit Hydrophobin B (H*B), aber auch mit Hydrophobin A (H*A) und eine anschließende MilchsäureBehandlung, in den tieferen Hautschichten (nach dem Stripping) zu einem niedrigeren pH-Wert der Haut führte als die Kontrolle ohne Hydrophobin. Dieses Ergebnis zeigt deutlich, dass Hydrophobin zu einer verbesserten Penetration der Milchsäure in die Haut führt, Hydrophobin also als Penetrationsenhancer für diesen Stoff dient.

## Patentansprüche

1. Pflanzenschutzmittel enthaltend Hydrophobin.

2. Pflanzenschutzmittel nach Anspruch 1 enthaltend mindestens einen Wirkstoff für den Pflanzenschutz.

3. Pflanzenschutzmittel nach Anspruch 1 enthaltend mindestens einen Wirkstoff für den Pflanzenschutz ausgewählt aus der Gruppe enthaltend Herbizide, Fungizide, Nematizide, Akarizide, Insektizide sowie Wirkstoffe, die das Pflanzenwachstum regulieren.

4. Pflanzenschutzmittel nach Anspruch 1 enthaltend mindestens ein Fungizid.

5. Pflanzenschutzmittel nach Anspruch 1 enthaltend mindestens ein Insektizid.

6. Pflanzenschutzmittel nach Anspruch 1 enthaltend Hydrophobin in einem Anteil von 0,000001 bis 10 Gew.-%.

7. Pflanzenschutzmittel nach Anspruch 1 **dadurch gekennzeichnet, dass** es sich um ein Wirkstoffkonzentrat handelt, welches den Wirkstoff in einer Menge von wenigstens 5 Gew.-% enthält.

8. Pflanzenschutzmittel nach Anspruch 1 **dadurch gekennzeichnet, dass** es sich um eine wässrige Zusammensetzung handelt.

9. Pflanzenschutzmittel nach Anspruch 1 **dadurch gekennzeichnet, dass** es sich um eine wässrige Zusammensetzung mit einem Anteil an organischen Lösungsmitteln von weniger als 10 Gew.-% handelt.

10. Pflanzenschutzmittel nach Anspruch 1 **dadurch gekennzeichnet, dass** es sich um eine lösungsmittelfreie Zusammensetzung handelt.

11. Pflanzenschutzmittel nach Anspruch 7 enthaltend den Wirkstoff in einer Menge von 5 bis 50 Gew.-%.

12. Verwendung von Hydrophobin in Pflanzenschutzmitteln.

13. Verwendung nach Anspruch 12 in Kombination mit mindestens einem Penetrationsverstärker.

14. Verwendung von Hydrophobin zur Herstellung wässriger Wirkstoffzusammen setzung von Insektiziden.

15. Verwendung von Hydrophobin zur Herstellung wässriger Wirkstoffzusammen setzung von Fipronil.
